(19) **Europäisches Patentamt**
**European Patent Office**
**Office européen des brevets**

(11) **EP 4 436 627 B1**

(12) **EUROPEAN PATENT SPECIFICATION**

(45) Date of publication and mention
of the grant of the patent:
**01.04.2026 Bulletin 2026/14**

(21) Application number: **22839883.0**

(22) Date of filing: **23.12.2022**

(51) International Patent Classification (IPC):
***A61M 1/16*** *(2006.01)* ***A61M 1/36*** *(2006.01)*

(52) Cooperative Patent Classification (CPC):
**A61M 1/36224; A61M 1/1605; A61M 1/1613;**
**A61M 1/1619; A61M 1/1696; A61M 1/3609;**
**A61M 1/361; A61M 1/3612; A61M 1/367;**
A61M 2205/3306; A61M 2205/3327;
A61M 2205/702; A61M 2230/20

(86) International application number:
**PCT/GB2022/053393**

(87) International publication number:
**WO 2023/118902 (29.06.2023 Gazette 2023/26)**

(54) **APPARATUS AND METHOD**

VORRICHTUNG UND VERFAHREN

APPAREIL ET PROCÉDÉ

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **24.12.2021 GB 202119007**

(43) Date of publication of application:
**02.10.2024 Bulletin 2024/40**

(73) Proprietor: **Kalium Health Ltd**
**Girton**
**Cambridgeshire CB3 0QH (GB)**

(72) Inventors:
• **HUTTER, Tanya**
**Cambridge House Camboro Business Park,**
**Oakington**
**Road Girton Cambridgeshire CB3 0QH (GB)**

• **COLLINGS, Thomas Stephen**
**Cambridge House Camboro Business Park,**
**Oakington**
**Road Girton Cambridgeshire CB3 0QH (GB)**

(74) Representative: **Appleyard Lees IP LLP**
**G Mill**
**Dean Clough Industrial Park**
**Halifax HX3 5AH (GB)**

(56) References cited:
**WO-A1-2020/223500** **US-A- 4 662 208**
**US-B2- 9 278 171**

## Description

## Field

The present invention relates to dialysis, for example haemodialysis.

## Background to the invention

Generally, healthy kidneys function to remove toxic waste products, such as urea, and balance pH and components including electrolytes, such as $Na^+$, $K^+$, $Ca^{2+}$, $Mg^{2+}$, $Cl^-$, $SO_4^{2-}$ and $PO_4^{3-}$. Patients, for example having end stage renal disease, are dependent on chronic dialysis treatment modalities, such as haemodialysis (also known as hemodialysis) or peritoneal dialysis.

During haemodialysis, for example, blood is taken from a patient through an intake needle (or catheter) which draws blood from an artery located in a specific access location (arm, thigh, subclavian, etc.). The blood is pumped via extracorporeal tubing and through a dialyser (also known as dialyzer). The blood passes through the dialyser in contact with a semi-permeable membrane, typically in a countercurrent direction to flow of a dialysate on the opposite side of the semi-permeable membrane. The dialyser replicates kidney function. The dialysed blood flows out of the dialyser via tubing and through another needle (or catheter) back into the patient. Treatment durations are typically 3 to 8 hours and may be repeated 3 to 7 times weekly.

In more detail, during haemodialysis, convection and diffusion between counterflowing blood and dialysate through the semi-permeable membrane of the dialyser replicate kidney function to re-establish blood plasma composition to normal values. Through convection, water, $Na^+$ and electrolytes are removed from the blood to the dialysate by hydraulic and osmotic pressure differences across the semi-permeable membrane while toxic waste products and other components are removed from the blood to the dialysate through diffusion through the semi-permeable membrane. Conversely, other components, such as bicarbonate, are transported through diffusion from the dialysate to the blood through the semi-permeable membrane.

However, during haemodialysis, an excess of electrolytes in the blood may be removed while in some cases, dialysis may result in insufficient removal of electrolytes. An excess of $Na^+$ in the blood may contribute to the patient feeling thirsty or may lead to hypertension while removal of too much $Na^+$ may result in a decline in blood volume, chest pain, nausea, vomiting, headache and/or muscle cramps. An excess of $K^+$ in the blood may lead to muscle pain, weakness and/or numbness while removal of too much $K^+$ may lead to heart rhythm disturbances. An excess of $Ca^{2+}$ in the blood can lead to vascular calcification while removal of too much $Ca^{2+}$ may lead to bone disorders and/or uncontrollable secondary parathyroid hormone (PTH) secretion.

Diffusion through the semi-permeable membrane is determined, at least in part, by characteristics of the semi-permeable membrane and respective concentration gradients of the components between the blood and the dialysate. While the characteristics of the semi-permeable membrane are predetermined, for example by the particular dialyser used, the respective concentration gradients of the components are typically not known a priori and/or may change during the haemodialysis, thereby adversely affecting an efficiency (e.g. rate, time to completion) and/or an effectiveness (e.g. extent or degree) of haemodialysis (i.e. re-establishing the blood plasma composition to normal values). For example, electrolyte composition in the blood is a highly dynamic function, dependent on many physiological and nutritional inputs, and subject to significant variability between patients. In most haemodialysis treatments, one or only a small number of dialysate compositions is available to treat patients, regardless of individual variations in electrolyte profiles that exist between patients or even between the same patient on different days. This "one-size-fits-all" approach to treatment may be reasonable for a majority of patients, but some patients do not tolerate it well.

Hence, there is a need to improve dialysis, for example haemodialysis and/or peritoneal dialysis.

Relevant prior art is for instance disclosed in documents US4662208 A and US9278171 B2.

## Summary of the Invention

It is one aim of the present invention, amongst others, to provide a haemodialysis machine which at least partially obviates or mitigates at least some of the disadvantages of the prior art, whether identified herein or elsewhere. For instance, it is an aim of embodiments of the invention to provide a haemodialysis machine that improves an efficiency and/or an effectiveness of haemodialysis, for example while improving patient safety and/or patient outcome. For example, it is an aim of embodiments of the invention to provide a haemodialysis machine that supplies a patient-specific (i.e. personalised) dialysate and/or supplies a dialysate adaptive to real-time changes in patient needs (e.g. personalised in real-time) between and/or even during haemodialysis treatments.

A first aspect (claimed) provides a haemodialysis machine comprising the technical features as defined in independent claim 1.

A second aspect (not claimed) provides an extracorporeal blood sensor unit comprising an analyte sensor. configured to provide signals responsive to sensing of analytes, and an auxiliary circuit, wherein the extracorporeal blood sensor unit is fluidically couplable to an extracorporeal blood circuit of a dialysis machine; wherein the extracorporeal blood sensor unit is arrangeable in:

a first arrangement, wherein the analyte sensor is in fluid communication with the extracorporeal blood circuit; and

a second arrangement, wherein the analyte sensor is in fluid communication with the auxiliary circuit; wherein the extracorporeal blood sensor unit is configured to transmit a first set of signals, including a first signal, to a control unit of the dialysis machine when the extracorporeal blood sensor unit is arranged in the first arrangement.

A third aspect (not claimed) provides a sensor unit comprising an analyte sensor, configured to provide signals responsive to sensing of analytes, and an auxiliary circuit, wherein the sensor unit is fluidically coupleable to an extracorporeal blood circuit or a dialysate circuit of a dialysis machine;
wherein the sensor unit is arrangeable in:

a first arrangement, wherein the analyte sensor is in fluid communication with the extracorporeal blood circuit or the dialysate circuit; and
optionally, a second arrangement, wherein the analyte sensor is in fluid communication with the auxiliary circuit;
wherein the sensor unit is configured to transmit a first set of signals, including a first signal, to a control unit of the dialysis machine when the sensor unit is arranged in the first arrangement.

A fourth aspect (not claimed) provides a method of controlling a haemodialysis machine, wherein the haemodialysis machine comprises:

an extracorporeal blood circuit, a dialyser, a dialysate circuit and a control unit, wherein the extracorporeal blood circuit and the dialysate circuit are respectively in fluid communication with the dialyser; and
a first extracorporeal blood sensor unit comprising an analyte sensor, configured to provide signals responsive to sensing of analytes, and an auxiliary circuit, wherein the first extracorporeal blood sensor unit is fluidically coupled to the extracorporeal blood circuit;
wherein the method comprises:
arranging the first extracorporeal blood sensor unit in:

a first arrangement, wherein the analyte sensor is in fluid communication with the extracorporeal blood circuit; and
a second arrangement, wherein the analyte sensor is in fluid communication with the auxiliary circuit; and
controlling, by the control unit, the dialysate circuit based, at least in part, on a first set of signals, including a first signal, received from the first extracorporeal blood sensor unit, provided by the analyte sensor when the first extracorporeal blood sensor unit is arranged in the first arrangement.

## Detailed Description of the Invention

According to the present invention there is provided a haemodialysis machine, as set forth in the appended claims. Also provided is an extracorporeal blood sensor unit (not claimed), a sensor unit (not claimed) and a method (not claimed).

Other features of the invention will be apparent from the dependent claims, and the description that follows.

### *Haemodialysis machine*

The first aspect (claimed) provides a haemodialysis machine comprising the technical features as defined in independent claim 1.

In this way, respective amounts and/or concentrations of analytes in the extracorporeal blood may be determined. Particularly, since the first extracorporeal blood sensor unit is arrangeable in the first arrangement (e.g. a sensing arrangement) and in the second arrangement (e.g. a maintenance arrangement), for example alternately, an accuracy and/or precision of the analyte sensor, for example with respect to the sensed analytes and hence of the sensing thereof, is improved, as described below, thereby improving an efficiency and/or an effectiveness of haemodialysis.

In this way, the dialysate circuit is controlled based, at least in part, on sensing of analytes, for example an amount and/or a concentration thereof, in the extracorporeal blood in the extracorporeal blood circuit. In this way, respective concentration gradients of the analytes may be determined, for example as described below, and the dialysate circuit controlled accordingly, thereby improving an efficiency (e.g. rate, time to completion) and/or an effectiveness (e.g. extent or degree) of haemodialysis (i.e. re-establishing the blood plasma composition to normal values), for example while improving patient safety and/or patient outcome.

By arranging the first extracorporeal blood sensor unit in the second arrangement, wherein the analyte sensor is in fluid communication with the auxiliary circuit, the analyte sensor may be buffered, washed or calibrated, for example, as described below. In this way, an accuracy and/or precision of the analyte sensor, for example with respect to the sensed analytes, is improved. In more detail, conventional sensing of analytes in blood may be degraded, for example by biofouling of the analyte due to the blood and/or drift, particularly over a duration of haemodialysis treatment. Additionally and/or alternatively, a relatively stationary layer of blood, that is replaced relatively slowly compared with the flowing extracorporeal blood, may be sensed by the analyte sensor, such that the conventional sensing is not reliable. In contrast, conventional sensing of analytes in dialysate may be relatively simple, given the relatively simple composition thereof.

The first aspect (claimed) provides the haemodialysis machine. Conventional haemodialysis machines are known. It should be understood that while the invention as claimed relates to the haemodialysis machine, the first aspect may similarly relate to a peritoneal dialysis machine (not claimed) mutatis mutandis and/or more generally, a dialysis machine (not claimed) mutatis mutandis.

The haemodialysis machine comprises the extracorporeal blood circuit, the dialyser, the dialysate circuit. The extracorporeal blood circuit and the dialysate circuit are respectively in fluid communication with the dialyser. Extracorporeal blood circuits, dialysers and dialysate circuits are known. Generally, extracorporeal blood flows, in use, through the extracorporeal blood circuit via the dialyser while dialysate counterflows, in use, through the dialysate circuit via the dialyser, to reestablish blood plasma composition to normal values.

Typically, dialysate is made by mixing two concentrate components, which may be provided as liquid or dry (powder) concentrates. The bicarbonate component typically contains sodium bicarbonate and optionally sodium chloride; the acid component typically contains chloride salts of sodium, potassium (optional), calcium, magnesium, acetate (or citrate), and glucose and/or dextrose (optional). These two components are mixed simultaneously together with purified water to make the dialysate. A dialysate proportioning pump ensures proper mixing. The relative amounts of water, bicarbonate, and acid components define the final dialysate composition. Alteration of the bicarbonate concentration may be achieved by changing the mixing ratio of water to bicarbonate. A variable sodium option allows the adaptation of the dialysate sodium concentration to the patient's needs. Glucose is usually added to prevent intradialytic hypoglycemia, but glucose concentrations of 200 mg/dl (11 mmol/l) may result in hyperglycemia and hyperinsulinemia. To avoid the need for large volumes of water, spent dialysate may be regenerated by sorbents, rather than diverted to waste. These systems may need as little as 6 litres of tap water for a regular dialysis treatment.

The composition of dialysate varies according to clinical needs. A standard dialysate aims to allow a net outflow of potassium from the blood, at a rate below that to create hypokalaemia, and a net inflow of calcium. A typical dialysate composition is an aqueous solution including (mmol / l): sodium 140.0; potassium 1.0; calcium 1.25; bicarbonate 34.0; magnesium 0.5; chloride 107.5; and glucose 5.5. Other dialysate compositions are known.

Typically, dialysate circuits include constant monitoring of conductivity and temperature of the dialysate. Typically, a proportioning unit (also known as a proportioning system or an electrolyte control unit) dilutes a concentrated dialysis solution with water, thereby providing the dialysate. If this proportioning system malfunctions, patient blood can be exposed to a hyperosmolar dialysis solution, resulting in hypernatremia, or a hypoosmolar dialysis solution, leading to hyponatremia and haemolysis. The primary solutes in the dialysis solution are electrolytes. Therefore, the concentration of the dialysis solution is reflected by the concentration of electrolytes and their electrical conductivity. Appropriate proportioning of water and the concentrated dialysis solution is typically monitored by a conductivity sensor measuring conductivity of the dialysate fed into the dialyser. Typically, a temperature sensor prevents complications related to warm dialysis solution. In most situations, the dialysate temperature is higher than that of the patient, resulting in the transfer of thermal energy into the patient. The energy transfer may prevent a rise in total peripheral resistance as fluid is removed by ultrafiltration (UF), resulting in hypotension during haemodialysis. A relatively cool dialysate (e.g. 35°C) may be uncomfortable for the patient and may be dangerous when the patient is unconscious. However, the use of relatively cool dialysate otherwise may have therapeutic value in preventing hypotension. Overheated dialysate (e.g. >42°C), however, can lead to haemolysis. If the conductivity or the temperature is outside the normal range, a bypass valve typically diverts the dialysate around the dialyser and directly to waste.

In one example, the dialysate circuit comprises a proportioning unit configured provide the dialysate, for example by diluting a concentrated dialysis solution with water thereby providing the dialysate and/or by dissolving one or more dialysis tablets in water thereby providing the dialysate, a dialysate pump (as described herein), a temperature sensor, a conductivity sensor and/or a dialysate regeneration unit.

In one example, the control unit is configured to adjust dialysate during dialysis for a patient, comprising:

subsequent to initiating dialysis for the patient, obtain, from the first extracorporeal blood sensor unit, a measurement of a concentration of the analyte in the extracorporeal blood;

determine whether the obtained measurement is within a predefined range;

in response to determining that the measurement is not within the predefined range, determine, based on the obtained measurement, at least one first adjustment value for adjusting a composition of the dialysate, wherein the composition of the dialysate is based on respective amounts of chemicals dispensed from a plurality of chemical sources; and

provide, based on the at least one first adjustment value, one or more first instructions to the proportioning unit to adjust the composition of the dialysate during dialysis for the patient by changing one or more of the respective amounts of chemicals dispensed from the plurality of chemical sources; and

control the proportioning unit to adjust the composition of the dialysate during dialysis based on the one or more first instructions.

In one example, the dialysate circuit comprises an electrolyte composition monitor, which may be provided by the control unit, and/or a dialysate mixing system, which may be provided by a proportioning unit.

In one example, during dialysis, an electrolyte composition monitor employs a dialysate mixing system to make an amount of dialysate on demand using, among other things, a plurality of chemical concentrates. The dialysate generated will have a formula, recipe, or prescription that differs from dialysate previously used during dialysis. The dialysate's formula will thus be adjusted during dialysis based on the electrolyte composition monitor detecting an elevated or depressed level of one or more electrolytes in a patient's blood during dialysis.

In one example, the dialysate used during dialysis is made in batches. Each batch follows a prescription, formula, or recipe chosen by the electrolyte composition monitor based on receiving electrolyte concentration levels from the patient's blood. The electrolyte composition monitor may continually adjust a next dialysate batch's recipe and task its dialysate mixing system to follow the prescribed recipe. For example, the dialysate mixing system may receive a recipe indicating particular chemical constituents and amounts of each chemical constituent to be included in the dialysate. Based on the prescription, the dialysate mixing system can determine, for example, a number of tablets, mass of powder, or volume of concentrated electrolyte solution required for each chemical constituent. Tablets, powders and/or concentrated electrolyte solutions, can be automatically dispensed and mixed with purified water, bicarbonate, and/or sodium chloride in a mixing chamber to produce the dialysate according to the desired dialysate recipe.

In one example, chemical constituents of the dialysate are provided, for example delivered, and stored in a tablet form or in a concentrated form, thus requiring minimal storage space and oversight. Mixing the dialysate in batches throughout dialysis suggests less storage space is required since the volume of dialysate made can be fully exhausted during a treatment session.

In one example, the control unit is configured to determine the chemical composition of each batch of dialysate. For example, a batch of dialysate may be 12 litres (L) and the chemical composition may include a plurality of chemical concentrates. The chemical concentrates may be liquid concentrates of varying viscosity and/or may be solid concentrates in the form of tablets, pills, or powders. The control unit may compute the chemical composition (e.g., an amount of each of the plurality of chemical concentrates such as a number of tablets) for each 12 L batch of dialysate based on a prescription issued by a physician/doctor.

In one example, the dialysate mixing system includes a dispenser and a mixing chamber 204. The dispenser can include chemical concentrates in chemical sources. The chemical concentrates are used as ingredients of the dialysate mixture. The chemical concentrates can be liquid concentrates of varying viscosity or can be solid concentrates in the form of tablets, pills, or powders. The chemical sources are containers that hold these chemical concentrates. Chemical sources can thus hold concentrates of potassium chloride (KCl), calcium chloride ($CaCl_2$), magnesium chloride ($MgCl_2$), citric acid, dextrose, sodium chloride (NaCl), sodium bicarbonate ($NaHCO_3$), acetic acid, glucose, and so on. Not all chemical concentrates available need to be used in a dialysate formula or recipe. For example, one recipe may only call for acetic acid, NaCl, $CaCl_2$, KCl, $MgCl_2$, and glucose. Another recipe may call for bicarbonate, NaCl, $CaCl_2$, KCl, and $MgCl_2$, without glucose.

In one example, the control unit is configured to determine adjustment values by unit increments. That is, after determining whether to increase or decrease a concentration of an electrolyte that is not within a predefined range, the control unit determines that the concentration of the electrolyte should be adjusted by a given unit. In an embodiment where chemical constituents of dialysate are adjusted by tablets, each unit represents an electrolyte concentration provided by a chemical concentrate's respective tablet. In an embodiment where chemical constituents of dialysate are adjusted by liquid concentrates, each unit represents an expected electrolyte concentration provided by opening its respective valve for a predetermined amount of time. Although one unit increments are described, adjustment values can be determined as multiple unit increments. For example, the control unit can determine that the concentration of the electrolyte that is not within its predefined range should be increased by three units which correspond to an amount of electrolytes expected from three tablets.

The haemodialysis machine comprises the control unit, for example a computer including a processor and a memory storing instructions which when executed by the processor, cause the control unit to control the haemodialysis machine as described herein.

The haemodialysis machine comprises the first extracorporeal blood sensor unit comprising the analyte sensor and the auxiliary circuit, as described below.

The first extracorporeal blood sensor unit is fluidically coupled to the extracorporeal blood circuit. In this way, the first extracorporeal blood sensor unit enables real-time control of the haemodialysis. In other words, the first extracorporeal blood sensor unit enables control of the haemodialysis within the timescale thereof i.e. by controlling the haemodialysis of the extracorporeal blood flowing through the extracorporeal blood circuit, such as by controlling (e.g. adjusting) a composition of the dialysate responsive to changes of the extracorporeal blood and/or the dialysate. Additionally and/or alternatively, the first extracorporeal blood sensor unit enables personalised control of the haemodialysis. In other words, the first extracorporeal blood sensor unit enables patient-specific control of the haemodialysis i.e. by controlling the haemodialysis of the extracorporeal blood flowing through the extracorporeal blood circuit, such as by controlling (e.g. adjusting) a composition of the dialysate specifically for the patient. In this way, the control may respond to a change in a concentration gradient of an analyte, for example due to a change in a concentration of the analyte in the extracorporeal blood and/or in the dialysate. Additionally and/or alternatively, the control may induce a change in a concentration gradients of an analyte, for example by inducing a change in a concentration of the analyte in the extracorporeal blood and/or in the dialysate.

In one example, the extracorporeal blood circuit is via the first extracorporeal blood sensor unit. In this way, the first extracorporeal blood sensor unit is in-line and/or on-line with the extracorporeal blood circuit such that, in use, the extracorporeal blood flows through the first extracorporeal blood sensor unit and through the dialyser. Generally, for in-line analysis, a sensor is disposed in the fluid flow, such that all of the fluid is sensed, while for on-line analysis, a sensor is disposed in the fluid flow in a bypass line, such that a bypass fraction of the fluid is sensed by the sensor in the bypass line before typically returning to the remainder of the flow. In contrast, for off-line analysis or for at-line analysis, a sample of the fluid is collected from the fluid flow and analysis conducted remotely.

In one example, the first extracorporeal blood sensor unit is fluidically coupled to the extracorporeal blood circuit via a divert (also known as a branch) line (i.e. a bifurcation). In this way, the first extracorporeal blood sensor unit is quasi on-line with the extracorporeal blood circuit such that, in use, a diverted fraction of the extracorporeal blood flows through the first extracorporeal blood sensor unit but does not flow through the dialyser and may instead be subsequently directed to waste, for example. In other words, a sensor is disposed in the fluid flow in a divert line, such that a diverted fraction of the fluid is sensed by the sensor in the divert line (analogously to a bypass line) before not returning to the remainder of the flow (in contrast to a bypass line). In this way, the diverted fraction of the extracorporeal blood is sensed on-line but contamination of the extracorporeal blood returned to the patient is eliminated. Additionally and/or alternatively, the analyte sensor may be exposed to other solutions, as described below, whilst introduction thereof to the extracorporeal blood returned to the patient is precluded.

In one example, the first extracorporeal blood sensor unit is fluidically coupled to the extracorporeal blood circuit upstream of the dialyser. In this way, the analyte sensor is disposed to provide signals responsive to sensing of analytes in the extracorporeal blood flowing from the patient, before dialysis thereof. By sensing the analytes in the extracorporeal blood before dialysis thereof, concentrations of analytes in the dialysate may be controlled reactively to changes in concentrations of analytes in the extracorporeal blood and/or proactively to induce changes in concentrations of analytes in the extracorporeal blood.

In one example, the first extracorporeal blood sensor unit is fluidically coupled to the extracorporeal blood circuit downstream of the dialyser. In this way, the analyte sensor is disposed to provide signals responsive to sensing of analytes in the extracorporeal blood returning to the patient, after dialysis thereof. By sensing the analytes in the extracorporeal blood after dialysis thereof, concentrations of analytes in the dialysate may be controlled reactively to induced changes in concentrations of analytes in the extracorporeal blood and/or proactively to induce further changes in concentrations of analytes in the extracorporeal blood. In this way, the dialysate may be personalised, for example in real-time.

In one example, the first extracorporeal blood sensor unit is configured to separate plasma from the extracorporeal blood (i.e. whole extracorporeal blood), for example by comprising filter paper and /or a set of bifurcated microchannels, including a first bifurcated microchannel (i.e. a microchannel is branched or divided, equally or non-equally, into two microchannels), and to direct the separated plasma to the analyte sensor. In this way, analytes are sensed in the separated plasma from the extracorporeal blood, rather than in the whole extracorporeal blood, thereby further improving an accuracy and/or precision of the analyte sensor, for example with respect to the sensed analytes, is improved.

Generally, flow of fluids, for example biological fluids such as blood, through a bifurcated microchannel (i.e. a microchannel is branched or divided, equally or non-equally, into two microchannels), may result in the Zweifach-Fung bifurcation law or effect. The Zweifach-Fung bifurcation law is an empirical law relating to the behaviour of deformable particles at a bifurcation in a channel. At such a bifurcation, a cell in a flowing fluid tends to be transported into the branched channel having the higher flowrate, providing that a dimension of the cell is comparable to a dimension of the branched channel. Although this law was first proposed in the context of micro-circulation in the human body, it may also apply to *in vitro* flow, such as in microchannels. Further, this bifurcation law may be extended to apply to populations of cells flowing in microchannels tens of microns wide.

Flow of fluids, for example biological fluids such as blood, through a constricted microchannel (i.e. a microchannel including a constriction) may result in a focusing effect. For example, a first liquid component may be focused towards the microchannel walls and/or a second liquid component may be focused away from the microchannel walls, or vice versa, due, at least in part, to the constriction. This focusing effect may be relatively small in relatively wider microchannels, for example a 200 $\mu$m wide microchannel, and/or at relatively higher flowrates. For example, cells such as red blood cells may be focused after flowing through a constriction, resulting in a substantially cell-free layer proximal the microchannel walls and a relatively cell-enriched stream centrally. In other words, a constriction before a bifurcation results in focusing before splitting of the flow. In this way, the cells are focused relatively more centrally in a channel and may tend to continue to flow therealong after the bifurcation while the plasma focused relatively more proximal the walls of the channel may tend to flow into the bifurcated conduit. In this way, separation of the plasma and the cells may be improved.

*Analyte sensor*

The first extracorporeal blood sensor unit comprises the analyte sensor. In this way, analytes in the extracorporeal blood, for example, may be sensed (i.e. detected), enabling estimation of respective levels, for example concentrations and/or amounts, thereof, for example quantitatively, semi-quantitatively and/or qualitatively.

In one example, the analyte sensor is configured to sense an electrolyte (i.e. an ion for example present in the extracorporeal blood), for example a particular electrolyte (i.e. selectively) or particular electrolytes. In one example, the electrolyte is $Na^+$, $K^+$, $Ca^{2+}$, $Mg^{2+}$, Cl-, $SO_4^{2-}$, $PO_4^{3-}$, or a mixture thereof. In one preferred example, the electrolyte is $Na^+$.

In one example, the analyte sensor comprises and/or is a conductivity sensor. Conductivity sensors are known. Generally, conductivity sensors are not ion-selective but may be used for urea clearance monitoring, for example, via an approximate calculation of ionic mass balance. In one example, the analyte sensor comprises a pair of conductivity sensors, respectively disposed the inlet and the output of the dialyser. Conductivity methods of measuring on-line ionic dialysate are known.

In one example, the analyte sensor comprises and/or is an ion-selective electrode (ISE) cell comprising a reference electrode, a set of ion-selective electrodes, including a first ion-selective electrode, and optionally a counter electrode. ISE cells are known. In contrast to conductivity sensors, ISEs are ion-selective and may be subject to crosstalk from other ions. For example, the concentration of $Na^+$ in dialysate is typically a factor of about 100 larger than that for $K^+$ and $Ca^{2+}$ and hence crosstalk calibration may be required. Conventionally, use of ISE cells as analyte sensors for in-line analysis or on-line analysis of dialysate has been unsuccessful due to membrane ageing and/or fouling, resulting in drift and requiring frequent recalibration which is conventionally not practical. Such problems are exacerbated by extracorporeal blood and hence conventionally, exclude use of ISE cells as analyte sensors for in-line analysis or on-line analysis of extracorporeal blood. However, the inventors have developed the auxiliary circuit to provide a solution to these problems, enabling washing and/or (re) calibration, for example, of the ISE cell during haemodialysis while using the ISE cell as an analyte sensor for on-line analysis of extracorporeal blood and optionally, of dialysate, as described below.

In one example, the analyte sensor comprises and/or is an optical sensor, for example a ultraviolet (UV) absorbance and near-infrared (NIR) absorbance spectrometer. Suitable spectrometers are known.

In one example, the analyte sensor comprises and/or is an atomic emission spectrometer, for example using flame photometry, such as based on fluorescent photoinduced electron transfer sensor or laser-induced breakdown spectroscopy. Suitable spectrometers are known.

In one example, the analyte sensor comprises and/or is a mass spectrometer, for example and inductively coupled plasma mass spectrometer. Suitable spectrometers are known.

In one example, the analyte sensor is replaceably (i.e. interchangeably) received in the first extracorporeal blood sensor unit. In this way, the analyte sensor may be removed and replaced, for example by a replacement analyte sensor. For example, the analyte sensor may be a disposable analyte sensor, such as a printed ISE cell provided on a substrate such as a card. In this way, the disposable analyte sensor may be replaced periodically, such as before or after each haemodialysis to avoid any potential contamination, or at daily or weekly intervals or on demand. In one example, the analyte sensor is slidably and replaceably received in the first extracorporeal blood sensor unit, for example by insertion and retraction, for example without requiring use of tools (i.e. tool-free).

In one example, the first extracorporeal blood sensor unit comprises a temperature sensor for temperature-controlled recalibration of the analyte sensor, to provide correct functioning of the analyte sensor. In one example, the first extracorporeal blood sensor unit comprises a flow sensor for flow-controlled recalibration of the analyte sensor, to provide correct functioning of the analyte sensor. In one example, the first extracorporeal blood sensor unit comprises a temperature controller, such as resistive heater or a Peltier element, for the analyte sensor. In this way, temperature of the analyte sensor may be actively controlled, thereby improving accuracy.

*Auxiliary circuit*

The first extracorporeal blood sensor unit comprises the auxiliary circuit. In this way, the analyte sensor may be buffered, washed or calibrated, for example, thereby enabling conditioning, reduction or elimination of biofouling and/or calibration or recalibration of the analyte sensor in use (i.e. during haemodialysis, for example throughout haemodialysis). In this way, an accuracy and/or precision of the analyte sensor, for example with respect to the sensed analytes, is improved. It should be understood that the auxiliary circuit is auxiliary or secondary (i.e. additional to, providing a supporting function) to the extracorporeal blood circuit and/or the dialysate circuit (i.e. the primary circuit). It should be understood that the auxiliary circuit may not form a closed-path circuit (c.f. the extracorporeal blood circuit and/or the dialysate circuit). It should be understood that generally, fluid in the auxiliary circuit does not return to the extracorporeal blood circuit and/or the dialysate circuit though may be configured to return to the extracorporeal blood circuit and/or the dialysate circuit.

In one example, the auxiliary circuit is configured to, for example selectively, provide a set of solutions and/or solvents, including a first solution for example a buffer, a wash, a calibrant and/or a diluent and/or a first solvent for example a polar solvent such as a polar aprotic solvent or a polar protic solvent as water, ethanol or acetone or a non-polar solvent such as a non-polar hydrocarbon, ether or chlorocarbon solvent, to the analyte sensor, in the second arrangement.

In one example, the auxiliary circuit comprises a pump, a valve and/or a set of solution and/or solvent reservoirs including a first solution and/or solvent reservoir. In one example, the pump comprises and/or is a dialysis pump, a diaphragm pump, a gear pump, a magnetically coupled pump or a syringe pump. Other pumps are known. In one example, the pump is provided by capillary action or wicking (i.e. passively), for example to an absorbent sponge and/or blotting paper, thereby providing a waste. In one example, the valve comprises and/or is a multi-port and/or multi-way switch valve, for example wherein respective ports are fluidically coupled to the extracorporeal blood circuit, to the set of solution reservoirs including the first solution reservoir, to the analyte sensor and optionally to a waste, wherein the first extracorporeal blood sensor unit is arranged to move between the first arrangement and the second arrangement(s) by switching the valve. Suitable valves are known. In one example, the first solution comprises and/or is a buffer, a wash, a calibrant and/or a diluent.

*Arrangements*

The first extracorporeal blood sensor unit is arrangeable, for example repeatedly, in the first arrangement and in the second arrangement. In other words, the first extracorporeal blood sensor unit is selectively arrangeable in the first arrangement or in the second arrangement. In this way, the first extracorporeal blood sensor unit may be arranged for sensing of analytes, by the analyte sensor, in the extracorporeal blood, or a portion thereof, and for washing or calibration of the analyte sensor, for example, respectively. Generally, the first arrangement corresponds with a sensing arrangement and the second arrangement corresponds with a maintenance arrangement.

In one example, the first extracorporeal blood sensor unit is configured to periodically, for example at a predetermined frequency and/or for a predetermined duration(s), or intermittently, for example for a predetermined duration, move between the first arrangement and the second arrangement(s). In this way, the first extracorporeal blood sensor unit may be arranged repeatedly in the first arrangement and in the second arrangement, for example alternately. In one example, the first extracorporeal blood sensor unit is configured to be arranged in the first arrangement for a first duration and in the second arrangement for a second duration, for example successively and/or repeatedly. It should be understood that the first duration corresponds with a sensing period, during which the sensor unit senses analytes present in the extracorporeal blood, notwithstanding that the sensing duration may be relatively shorter than the first duration. It should be understood that the second duration corresponds with a maintenance period, during which the sensor unit is maintained, for example as described below. In one example, the first duration is in a range from 1 s to 600 s, preferably in a range from 10 s to 300 s, more preferably in a range from 30 s to 150 s. In one example, the second duration is in a range from 1 s to 600 s, preferably in a range from 10 s to 300 s, more preferably in a range from 30 s to 150 s. In one example, a ratio of the first duration to the second duration is in a range from 10 : 1 to 1 : 20, preferably in a range from 5 : 1 to 1: 10, more preferably in a range from 1 : 1 to 1 : 5. For example, the first extracorporeal blood sensor unit may be configured to alternate between a 1 minute (60 s) sensing period and a 5 minute (300 s) maintenance period during dialysis and/or between a 1 minute (60 s) sensing period and a 1 minute (60 s) maintenance period during rapid sampling. In one example, the first extracorporeal blood sensor unit is configured to alternately, for example periodically or intermittently, move between the first arrangement and the second arrangement. In one example, the control unit is configured to control the first extracorporeal blood sensor unit to move between the first arrangement and the second arrangement, for example periodically and/or intermittently, for example in response to a control signal transmitted by the control unit to the first extracorporeal blood sensor unit. In one example, the control unit is configured to transmit the control signal reactively and/or proactively with respect to an event, for example as described below, for example to respond to a change in a concentration gradient of an analyte and/or to induce a change in a concentration gradient of an analyte.

*First arrangement*

The analyte sensor is in fluid communication with the extracorporeal blood circuit, when the first extracorporeal blood sensor unit is arranged in the first arrangement. It should be understood that, in use, the analyte sensor of the first extracorporeal blood sensor unit is exposed to the extracorporeal blood, or a portion thereof, from the extracorporeal blood circuit when the first extracorporeal blood sensor unit is arranged in the first arrangement. In this way, analytes in the extracorporeal blood may be sensed.

In one example, the analyte sensor is in fluid communication with only the extracorporeal blood circuit in the first configuration. In this way, analytes in only the extracorporeal blood may be sensed.

In one example, the analyte sensor is in flowing fluid, for example continuously or intermittently flowing, communication with the extracorporeal blood circuit in the first arrangement. In this way, analytes in the flowing extracorporeal blood may be sensed, for example synchronously and/or simultaneously with haemodialysis of flowing extracorporeal blood in the extracorporeal blood circuit.

*Second arrangement*

The analyte sensor is in fluid communication with the auxiliary circuit, when the first extracorporeal blood sensor unit is arranged in the second arrangement. It should be understood that, in use, the analyte sensor of the first extracorporeal blood sensor unit is generally exposed to a fluid from the auxiliary circuit when the first extracorporeal blood sensor unit is arranged in the second arrangement.

In one example, the analyte sensor is in fluid communication with only the auxiliary circuit in the second configuration. In this way, the analyte sensor may be buffered, washed or calibrated, in the absence of extracorporeal blood, for example.

*Third arrangement*

In one example, the first extracorporeal blood sensor unit is arrangeable in:
a third arrangement, wherein the analyte sensor is in fluid communication with the extracorporeal blood circuit and with the auxiliary circuit.

In this way, a calibration standard and/or a diluent, for example, may be included in the extracorporeal blood and the extracorporeal blood, having the calibration standard and/or the diluent, for example, included therein sensed using the analyte sensor. In this way, an accuracy and/or precision of the analyte sensor, for example with respect to the sensed analytes, may be further improved.

*Control unit*

The control unit is configured to control the dialysate circuit based, at least in part, on the first set of signals, including the first signal, received from the first extracorporeal blood sensor unit, provided by the analyte sensor when the first extracorporeal blood sensor unit is arranged in the first arrangement. In this way, the control unit provides feedback control of the dialysate circuit in real time. In this way, the control unit may control the dialysate circuit to respond to a change in a concentration gradient of an analyte, for example due to a change in a concentration of the analyte in the extracorporeal blood and/or in the dialysate, by changing a concentration of the analyte in the dialysate and/or a flow rate. Additionally and/or alternatively, the control unit may control the dialysate circuit to induce a change in a concentration gradient of an analyte, for example by inducing a change in a concentration of the analyte in the extracorporeal blood and/or in the dialysate, by changing a concentration of the analyte in the dialysate and/or a flow rate. It should be understood that the control unit is communicatively coupled, for example unidirectionally or bidirectionally, for example wired or wirelessly, to at least the first extracorporeal blood sensor unit and the dialysate circuit and as required to perform the functions described herein.

In one example, the dialysate circuit comprises a dialysate pump and the control unit is configured to control a pumping speed of the dialysate pump based, at least in part, on the first set of signals.

In one example, the dialysate circuit comprises an electrolyte control unit (also known as a proportioning unit) for controlling a concentration of an electrolyte, such as $Na^+$, $K^+$ and $Ca^{2+}$, in the dialysate, for example upstream of the dialyser and downstream of a dialysate regeneration unit. In one example, the dialysate circuit comprises a by-pass loop, regulated by a by-pass regulator, for example a pinch valve, on/off valve, or a valve with a range of open conditions such as a needle valve, to determine an amount of dialysate that flows through the electrolyte control unit.

In one example, the control unit is configured to control the auxiliary circuit based, at least in part, on a second set of signals, including a first signal, received from the extracorporeal blood sensor unit, provided by the analyte sensor when the first extracorporeal blood sensor unit is arranged in the second arrangement. In this way, the control unit provides feedback control of the auxiliary circuit, for example by controlling conditioning, calibration and/or recalibration of the analyte sensor.

In one example, the control unit is configured to condition, calibrate and/or recalibrate the analyte sensor, when the first extracorporeal blood sensor unit is arranged in the second arrangement.

The control unit is configured to control the dialysate circuit based, at least in part, a rate of change of the first set of signals, including the first signal. In this way, the dialysate circuit is controlled according to the rate of change of the first set of signals, for example according to a rate of change of a level of an analyte.

In one example, the control unit is configured to control the first extracorporeal blood sensor unit to perform one or more checks, for example upon start up (i.e. during initialization) and/or periodically, for example to verify operation of the analyte sensor such as by sensing quality control check solutions. In this way, operation of the analyte sensor is verified and in turn, operation of the haemodialysis machine may be verified therefrom.

In one example, the control unit is configured to control, for example adjust, a composition of the dialysate, for example during haemodialysis of a patient, for example by: subsequent to initiating haemodialysis of the patient, obtaining, by the control unit from the first extracorporeal blood sensor unit, a measurement of a concentration of an analyte, for example an electrolyte, in the patient's blood; determining, by the control unit, whether the obtained measurement is within a predefined range; in response to determining that the measurement is not within the predefined range, determining, by the control unit and based on the obtained measurement, at least one first adjustment value for adjusting a composition of the dialysate, wherein the composition of the dialysate is based on respective amounts of chemicals dispensed from a plurality of chemical sources by the dialysate circuit; and controlling, by the control unit and based on the at least one first adjustment value, the dialysate circuit to adjust the composition of the dialysate during haemodialysis for the patient by changing one or more of the respective amounts of chemicals dispensed from the plurality of chemical sources.

In one example, the control unit is configured to control, based on the at least one first adjustment value, the dialysate circuit to adjust the composition of the dialysate by providing one or more first instructions to direct the dialysate circuit to adjust the composition of the dialysate. In one example, the control unit is configured to, subsequent to adjusting the composition of the dialysate during haemodialysis based on the at least one first adjustment value, obtain a second measurement of the concentration of the analyte, for example an electrolyte, in the extracorporeal blood from the first extracorporeal blood sensor units; determine whether the at least one first adjustment value caused the second measurement to be within the predefined range; and in response to determining that the second measurement is not within the predefined range, control the dialysate circuit to adjust the composition of the dialysate during haemodialysis based on the second measurement.

In one example, the control unit is configured to control the dialysate circuit, in response to determining that the second measurement is within the predefined range, maintain the composition of the dialysate during haemodialysis.

In one example, the control unit is configured to obtain a second measurement of a second concentration of a second analyte, for example an electrolyte, in the patient's blood, wherein the second analyte, for example an electrolyte, and the first analyte, for example an electrolyte, are different analytes, for example different electrolytes; determine whether the second measurement is within a second predefined range; in response to determining that the second measurement is not within the second predefined range, determine, based on the second measurement, at least one second adjustment value for adjusting the composition of the dialysate, and control the dialysate circuit to adjust the composition of the dialysate based on the at least one first adjustment value and the at least one second adjustment value.

In one example, the control unit is configured to control the dialysate circuit to adjust the composition of the dialysate by generating actuating signals for changing the composition of the dialysate during haemodialysis based on the at least one first adjustment value; and provide the actuating signals to one or more actuators of the dialysate circuit to change proportions of the respective amounts of chemicals dispensed from the plurality of chemical sources.

In one example, the control unit is configured to control the dialysate circuit based on determining the at least one first adjustment value the analyte, for example an electrolyte, is an increase, by generating a first actuating signal for dispensing a higher proportion of a respective chemical of a respective chemical source of the plurality of chemical sources; and based on determining the at least one first adjustment value the analyte, for example an electrolyte, is a decrease, by generating a second actuating signal for dispensing a lower proportion of the respective chemical of the respective chemical source of the plurality of chemical sources.

*Second extracorporeal blood sensor unit*

In one example, the haemodialysis machine comprises a second extracorporeal blood sensor unit fluidically coupled to the extracorporeal blood circuit, for example upstream or downstream of the dialyser. The second extracorporeal blood sensor unit maybe generally as described with respect to the first extracorporeal blood sensor unit. In this way, analytes in the extracorporeal blood flowing from the patient before dialysis and in the extracorporeal blood returning to the patient after dialysis may be sensed, for example simultaneously, by different analyte sensors. In one example, the auxiliary circuit of the first extracorporeal blood sensor unit is common to both (i.e. shared by) the first extracorporeal blood sensor unit and the second extracorporeal blood sensor unit (i.e. a single auxiliary circuit). In this way, a cost and/or complexity of the haemodialysis machine is reduced. In one example, the analyte sensor of the first extracorporeal blood sensor unit is common to both (i.e. shared by) the first extracorporeal blood sensor unit and the second extracorporeal blood sensor unit (i.e. a single analyte sensor). In this way, analytes in the extracorporeal blood flowing from the patient before dialysis and in the extracorporeal blood returning to the patient after dialysis may be sensed, for example alternately, by the same analyte sensor, thereby avoiding cross-calibration of two analyte sensors.

In one example, the first extracorporeal blood sensor unit is fluidically coupled to the extracorporeal blood circuit upstream of the dialyser and the second extracorporeal blood sensor unit is fluidically coupled to the extracorporeal blood circuit downstream of the dialyser.

Additionally, alternatively and/or equivalently, in one example, the first extracorporeal blood sensor unit is fluidically coupled to the extracorporeal blood circuit upstream of the dialyser and downstream of the dialyser. In this way, analytes in the extracorporeal blood flowing from the patient before dialysis and in the extracorporeal blood returning to the patient after dialysis may be sensed, for example alternately, by the same analyte sensor.

In one example, the control unit is configured to control the dialysate circuit based, at least in part, on a difference between the first set of signals received from the first extracorporeal blood sensor unit and a second set of signals, including a first signal, for example received from the second extracorporeal blood sensor unit or the first extracorporeal blood sensor unit. In this way, the dialysate circuit is controlled according to the difference, for example according to a difference of a level of an analyte in the extracorporeal blood before and after dialysis.

In one example, the control unit comprises a trained machine learning algorithm, wherein the trained machine learning algorithm is trained to control the dialysate circuit based, at least in part, using the first set of signals received from the first extracorporeal blood sensor unit and optionally, a second set of signals, including a first signal, for example received from the second extracorporeal blood sensor unit or the first extracorporeal blood sensor unit. In one example, the trained machine learning algorithm is trained to control the dialysate circuit based, at least in part, using one or more sets of signals, including a first signal, received from a first dialysate sensor unit and/or a second dialysate sensor unit, as described herein. In one example, the machine learning algorithm is trained to infer a composition of the extracorporeal blood upstream and/or downstream of the dialyser and/or a composition of the dialysate upstream and/or downstream of the dialyser, using signals received from the first set of signals received from the first extracorporeal blood sensor unit. In this way, the dialysate circuit is controlled according to inferred compositions.

*Dialysate sensor unit*

In one example, the haemodialysis machine comprises a first dialysate sensor unit comprising an analyte sensor, wherein the first dialysate sensor unit is fluidically coupled to the dialysate circuit. The first dialysate sensor unit may be as described with respect to the first extracorporeal blood sensor unit mutatis mutandis. In one example, the first dialysate sensor unit comprises an auxiliary circuit, for example as described with respect to the first extracorporeal blood sensor unit mutatis mutandis. In this way, analytes in the dialysate may be sensed (i.e. detected), enabling estimation of respective levels, for example concentrations and/or amounts, thereof, for example quantitatively, semi-quantitatively and/or qualitatively. In this way, analytes in the dialysate may be controlled responsively (i.e. feedback control), for example by the control unit.

In one example, the first dialysate sensor unit is fluidically coupled to the dialysate circuit upstream of the dialyser. In this way, analytes in the dialysate before dialysis may be sensed (i.e. detected), enabling estimation of respective levels, for example concentrations and/or amounts, thereof, for example quantitatively, semi-quantitatively and/or qualitatively.

In one example, the haemodialysis machine comprises a second dialysate sensor unit comprising an analyte sensor, wherein the second dialysate sensor unit is fluidically coupled to the dialysate circuit downstream of the dialyser. In this way, analytes in the dialysate after dialysis may be sensed (i.e. detected), enabling estimation of respective levels, for example concentrations and/or amounts, thereof, for example quantitatively, semi-quantitatively and/or qualitatively.

In one example, the haemodialysis machine comprises a third dialysate sensor unit comprising an analyte sensor, wherein the third dialysate sensor unit is fluidically coupled to a tank containing dialysate. In this way, analytes in the dialysate may be sensed (i.e. detected), enabling estimation of respective levels, for example concentrations and/or amounts, thereof, for example quantitatively, semi-quantitatively and/or qualitatively.

In one example, the control unit is configured to control the dialysate circuit based, at least in part, on a difference between a first set of signals, including a first signal, received from the first dialysate sensor unit and a second set of signals, including a first signal, received from the second dialysate sensor unit. In this way, a level, for example a concentration and/or an amount, of an analyte, for example an electrolyte such as $Na^+$, $K^+$, $Ca^{2+}$ in the dialysate may be controlled, for example optimised.

*Multiplexed sensor unit*

In one example, the first extracorporeal blood sensor unit comprises and/or is a multiplexed sensor unit, wherein the multiplexed sensor unit is arrangeable in:

the first arrangement, wherein the analyte sensor is in fluid communication with the extracorporeal blood circuit upstream of the dialyser;
the second arrangement, wherein the analyte sensor is in fluid communication with the auxiliary circuit;
optionally, a third arrangement, wherein the analyte sensor is in fluid communication with the extracorporeal blood circuit downstream of the dialyser;
optionally, a fourth arrangement, wherein the analyte sensor is in fluid communication with the dialysate circuit upstream of the dialyser; and
optionally, a fifth arrangement, wherein the analyte sensor is in fluid communication with the dialysate circuit downstream of the dialyser.

*Extracorporeal blood sensor unit*

The second aspect (not claimed) provides an extracorporeal blood sensor unit comprising an analyte sensor. configured to provide signals responsive to sensing of analytes, and an auxiliary circuit, wherein the extracorporeal blood sensor unit is fluidically couplable to an extracorporeal blood circuit of a dialysis machine; wherein the extracorporeal blood sensor unit is arrangeable in:

> a first arrangement, wherein the analyte sensor is in fluid communication with the extracorporeal blood circuit; and
> a second arrangement, wherein the analyte sensor is in fluid communication with the auxiliary circuit; wherein the extracorporeal blood sensor unit is configured to transmit a first set of signals, including a first signal, to a control unit of the dialysis machine when the extracorporeal blood sensor unit is arranged in the first arrangement.

The extracorporeal blood sensor unit may be as described with respect to the first aspect.

*Sensor unit*

The third aspect (not claimed) provides a sensor unit comprising an analyte sensor, configured to provide signals responsive to sensing of analytes, and an auxiliary circuit, wherein the sensor unit is fluidically couplable to an extracorporeal blood circuit or a dialysate circuit of a dialysis machine; wherein the sensor unit is arrangeable in:

> a first arrangement, wherein the analyte sensor is in fluid communication with the extracorporeal blood circuit or the dialysate circuit; and
> optionally, a second arrangement, wherein the analyte sensor is in fluid communication with the auxiliary circuit; wherein the sensor unit is configured to transmit a first set of signals, including a first signal, to a control unit of the dialysis machine when the sensor unit is arranged in the first arrangement.

The sensor unit may be as described with respect to the extracorporeal blood sensor unit described with respect to the first aspect, mutatis mutandis.

*Method*

The fourth aspect (not claimed) provides a method of controlling a haemodialysis machine, wherein the haemodialysis machine comprises:

> an extracorporeal blood circuit, a dialyser, a dialysate circuit and a control unit, wherein the extracorporeal blood circuit and the dialysate circuit are respectively in fluid communication with the dialyser; and
> a first extracorporeal blood sensor unit comprising an analyte sensor, configured to provide signals responsive to sensing of analytes, and an auxiliary circuit, wherein the first extracorporeal blood sensor unit is fluidically coupled to the extracorporeal blood circuit;
> wherein the method comprises:
> arranging the first extracorporeal blood sensor unit in:

>> a first arrangement, wherein the analyte sensor is in fluid communication with the extracorporeal blood circuit; and
>> a second arrangement, wherein the analyte sensor is in fluid communication with the auxiliary circuit; and
>> controlling, by the control unit, the dialysate circuit based, at least in part, on a first set of signals, including a first signal, received from the first extracorporeal blood sensor unit, provided by the analyte sensor when the first extracorporeal blood sensor unit is arranged in the first arrangement.

The haemodialysis machine may be as described with respect to the first aspect. The method may include any of the steps described with respect to the first aspect.

*Definitions*

Throughout this specification, the term "comprising" or "comprises" means including the component(s) specified but not to the exclusion of the presence of other components. The term "consisting essentially of" or "consists essentially of" means including the components specified but excluding other components except for materials present as impurities, unavoidable materials present as a result of processes used to provide the components, and components added for a purpose other than achieving the technical effect of the present disclosure, such as colourants, and the like.

The term "consisting of" or "consists of" means including the components specified but excluding other components.

Whenever appropriate, depending upon the context, the use of the term "comprises" or "comprising" may also be taken to include the meaning "consists essentially of" or "consisting essentially of", and also may also be taken to include the meaning "consists of" or "consisting of".

The optional features set out herein may be used either individually or in combination with each other where appropriate.

## Brief description of the drawings

For a better understanding of the invention, and to show how exemplary embodiments of the same may be brought into effect, reference will be made, by way of example only, to the accompanying diagrammatic Figures, in which:

Figure 1 schematically depicts a haemodialysis machine according to an exemplary embodiment, arranged in a first arrangement;

Figure 2 schematically depicts the haemodialysis machine of Figure 1, arranged in a second arrangement;

Figure 3 schematically depicts a haemodialysis machine according to an exemplary embodiment, arranged in a first arrangement;

Figure 4 schematically depicts the haemodialysis machine of Figure 2, arranged in a second arrangement;

Figure 5 schematically depicts a haemodialysis machine according to an exemplary embodiment, arranged in a first arrangement;

Figure 6 schematically depicts a method according to an exemplary embodiment;

Figure 7 schematically depicts a sensor unit according to an exemplary embodiment;

Figure 8A is a graph of Delta E (mV) as a function of log $[K^+]$ (M) for the sensor unit of Figure 7; and Figure 8B is a graph of Potential (V) (smoothed) for stepping down $[K^+]$ as a function of time;

Figure 9 schematically depicts a sensor unit according to an exemplary embodiment;

Figure 10A is a graph of Delta E (mV) as a function of log $[K^+]$ (M) for the sensor unit of Figure 9; and Figure 10B is a graph of Potential (V) (smoothed) for stepping down $[K^+]$ as a function of time;

Figure 11A is a graph of Delta E (mV) as a function of log $[K^+]$ (M) for the sensor unit of Figure 9; and Figure 11B is a graph of Potential (V) (smoother) for stepping up $[K^+]$ as a function of time;

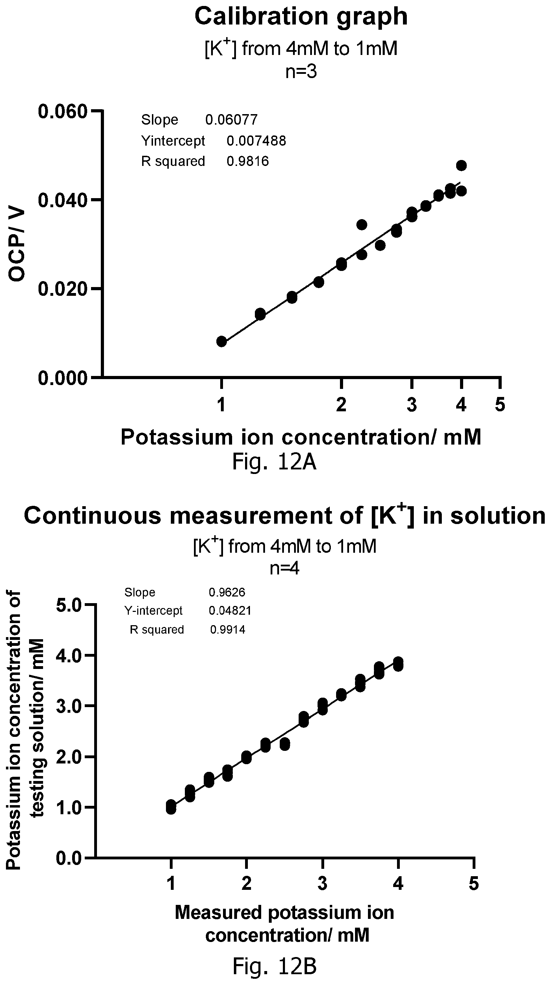

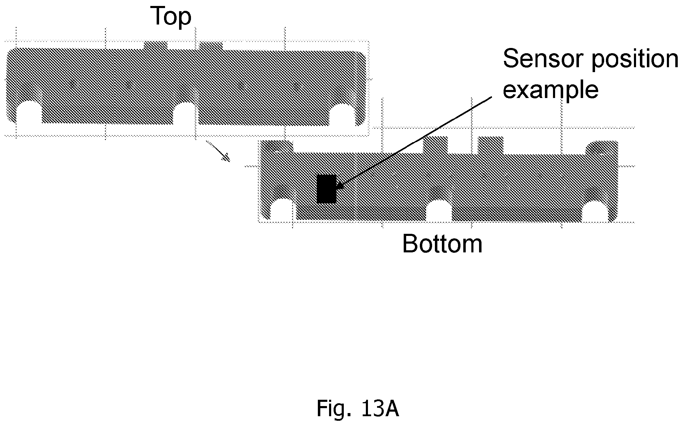

Figure 12A is a semi-log calibration graph of measured OCP (V) as a function of $[K^+]$ (mM) for buffered calibration solutions, for $[K^+]$ from 4 mM to 1 mM at 0.25 mM intervals and measured for 3 replicates at each concentration using the flow cell of Figures 13A to 13C (x-axis scale is a log10 scale); and Figure 12B is a graph of actual (i.e. prepared concentration of testing solution) $[K^+]$ (mM) as a function of measured $[K^+]$ (mM), for $[K^+]$ from 4 mM to 1 mM at 0.25 mM intervals and measured for 4 replicates at each concentration using the flow cell of Figures 13A to 13C, by continuous measurement;

Figure 13A is a CAD exploded, perspective view from above of a flow cell for four sensors, showing one sensor in position; Figure 13B is a CAD exploded, perspective view from above of the flow cell; and Figure 13C is a CAD partially-assembled, perspective view from above the flow cell (hinge rod to be inserted);

Figure 14A and Figure 14B are graphs of OCP (V) as a function of $[K^+]$ for $K^+$ in blood, for $[K^+]$ from 6.8 mM to 3.8 mM for 3 concentrations and measured for 2 replicates at each concentration using the flow cell of Figures 15A to 15C, by continuous measurement; Figure 14A is a graph of OCP (V) as a function of log $[K^+]$; and Figure 14B is a semi-log graph of the same data as Figure 14B of OCP (V) as a function of $[K^+]$ (mM) (x-axis scale is a log10 scale);

Figure 15A is a CAD exploded plan view of a flow cell for two sensors; Figure 15B is a CAD exploded view from above of the flow cell; and Figure 15C is a CAD exploded view from above of the flow cell; and

Figure 16 schematically depicts an exploded, perspective view of an ion-selective electrode cell (i.e. a sensor) for use in the flow cell of Figures 13A and 13B and the flow cell of Figures 15A to 15C.

## Detailed Description of the Drawings

Like reference signs denote like features.

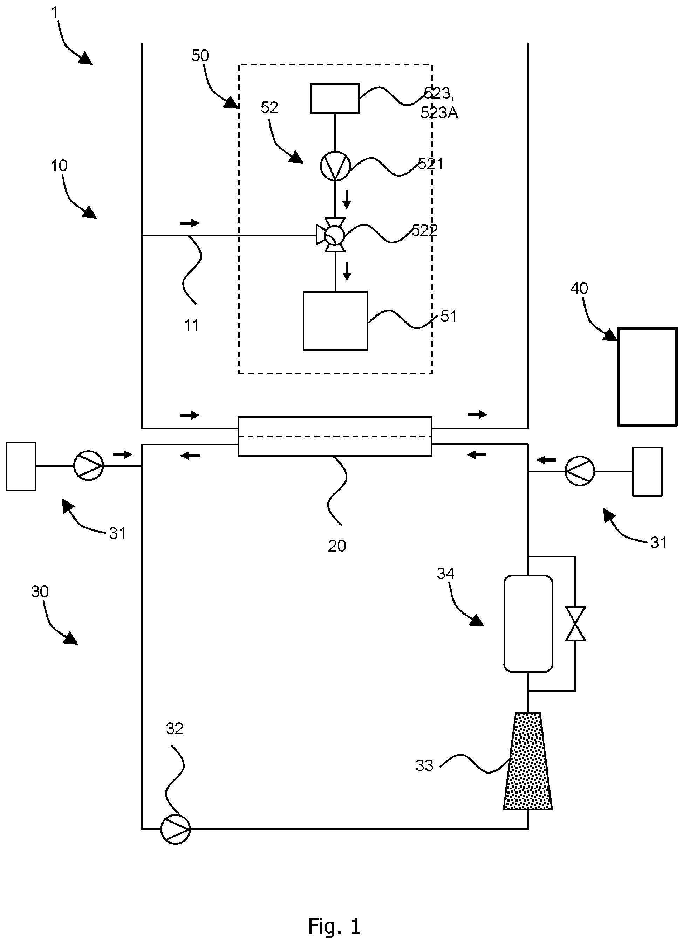

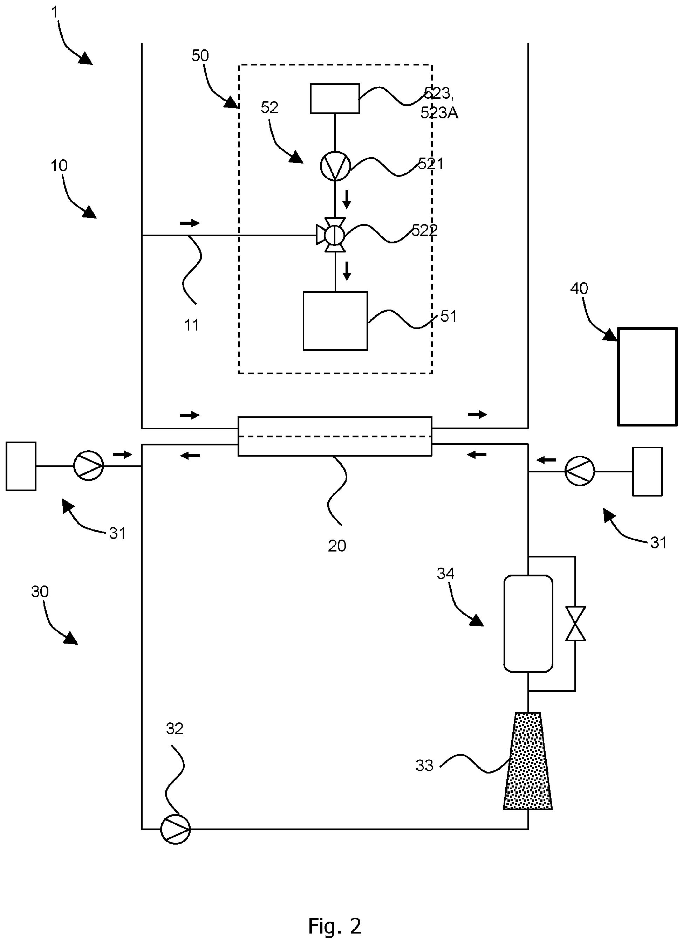

Figure 1 schematically depicts a haemodialysis machine 1 according to an exemplary embodiment, arranged in a first arrangement. Figure 2 schematically depicts the haemodialysis machine 1 of Figure 1, arranged in a second arrangement.
The haemodialysis machine 1 comprises:

an extracorporeal blood circuit 10, a dialyser 20, a dialysate circuit 30 and a control unit 40, wherein the extracorporeal blood circuit 10 and the dialysate circuit 30 are respectively in fluid communication with the dialyser 20; and
a first extracorporeal blood sensor unit 50 comprising an analyte sensor 51, configured to provide signals responsive to sensing of analytes, and an auxiliary circuit 52, wherein the first extracorporeal blood sensor unit 50 is fluidically coupled to the extracorporeal blood circuit 10;
wherein the first extracorporeal blood sensor unit 50 is arrangeable in:

a first arrangement, wherein the analyte sensor 51 is in fluid communication with the extracorporeal blood circuit 10; and

a second arrangement, wherein the analyte sensor 51 is in fluid communication with the auxiliary circuit 52;

optionally, wherein the control unit 40 is configured to control the dialysate circuit 30 based, at least in part, on a first set of signals, including a first signal, received from the first extracorporeal blood sensor unit 50, provided by the analyte sensor 51 when the first extracorporeal blood sensor unit 50 is arranged in the first arrangement.

The composition of dialysate varies according to clinical needs. A standard dialysate aims to allow a net outflow of potassium from the blood, at a rate below that to create hypokalaemia, and a net inflow of calcium. A typical dialysate composition is an aqueous solution including (mmol / l): sodium 140.0; potassium 1.0; calcium 1.25; bicarbonate 34.0; magnesium 0.5; chloride 107.5; and glucose 5.5. Other dialysate compositions are known.

In this example, the control unit 40 comprises a computer including a processor and a memory storing instructions which when executed by the processor, cause the control unit 40 to control the haemodialysis machine 1 as described herein.

In this example, the first extracorporeal blood sensor unit 50 is fluidically coupled to the extracorporeal blood circuit 10 via a divert 11 (also known as a branch) line (i.e. a bifurcation).

In this example, the first extracorporeal blood sensor unit 50 is fluidically coupled to the extracorporeal blood circuit 10 upstream of the dialyser 20.

In this example, the analyte sensor 51 is configured to sense an electrolyte (i.e. an ion for example present in the extracorporeal blood), for example a particular electrolyte (i.e. selectively) or particular electrolytes. In this example, the electrolyte is $Na^+$, $K^+$, $Ca^{2+}$, $Mg^{2+}$, Cl-, $SO_4^{2-}$, $PO_4^{3-}$ or a mixture thereof. In one preferred example, the electrolyte is $Na^+$.

In this example, the analyte sensor 51 is an ion-selective electrode (ISE) cell comprising a reference electrode, a set of ion-selective electrodes, including a first ion-selective electrode, and optionally a counter electrode. ISE cells are known.

In this example, the analyte sensor 51 is replaceably (i.e. interchangeably) received in the first extracorporeal blood sensor unit 50. In this example, the analyte sensor 51 is slidably and replaceably received in the first extracorporeal blood sensor unit 50, for example by insertion and retraction, for example without requiring use of tools (i.e. tool-free).

In this example, the auxiliary circuit 52 is configured to, for example selectively, provide a set of solutions, including a first solution for example a buffer, a wash, a calibrant and/or a diluent, to the analyte sensor 51, in the second arrangement.

In this example, the auxiliary circuit 52 comprises a pump 521, a valve 522 and a set of solution reservoirs 523 including a first solution reservoir 523A. In this example, the valve 522 is a multi-port and/or multi-way switch valve, for example wherein respective ports are fluidically coupled to the extracorporeal blood circuit 10, to the set of solution reservoirs including the first solution reservoir, to the analyte sensor 51 and optionally to a waste, wherein the first extracorporeal blood sensor unit 50 is arranged to move between the first arrangement and the second arrangement(s) by switching the valve.

In this example, the first extracorporeal blood sensor unit 50 is configured to periodically, for example at a predetermined frequency and/or for a predetermined duration(s), or intermittently, for example for a predetermined duration, move between the first arrangement and the second arrangement(s). In this example, the first extracorporeal blood sensor unit 50 is configured to be arranged in the first arrangement for a first duration and in the second arrangement for a second duration, for example successively and/or repeatedly. In this example, the first duration is in a range from 30 s to 150 s. In this example, the second duration is in a range from 30 s to 150 s. In this example, the first extracorporeal blood sensor unit 50 is configured to alternately, for example periodically or intermittently, move between the first arrangement and the second arrangement.

In this example, the analyte sensor 51 is in fluid communication with only the extracorporeal blood circuit 10 in the first configuration. In this way, analytes in only the extracorporeal blood may be sensed.

In this example, the analyte sensor 51 is in flowing fluid, for example continuously or intermittently flowing, communication with the extracorporeal blood circuit 10 in the first arrangement.

In this example, the analyte sensor 51 is in fluid communication with only the auxiliary circuit 52 in the second configuration. In this way, the analyte sensor 51 may be buffered, washed or calibrated, in the absence of extracorporeal blood, for example.

In this example, the dialysate circuit 30 comprises a dialysate pump 32 and the control unit 40 is configured to control a pumping speed of the dialysate pump 32 based, at least in part, on the first set of signals.

In this example, the dialysate circuit 30 comprises a proportioning unit 31 for controlling a concentration of an electrolyte, such as $Na^+$, $K^+$ and $Ca^{2+}$, in the dialysate, for example upstream of the dialyser 20 and downstream of a dialysate regeneration unit 33. In this example, the dialysate circuit 30 comprises a by-pass loop 34, regulated by a by-pass regulator, for example a pinch valve, on/off valve, or a valve with a range of open conditions such as a needle valve, to determine an amount of dialysate that flows through the electrolyte control unit 40.

In this example, the control unit 40 is configured to control the auxiliary circuit 52 based, at least in part, on a second set of signals, including a first signal, received from the extracorporeal blood sensor unit, provided by the analyte sensor 51 when the first extracorporeal blood sensor unit 50 is arranged in the second arrangement. In this way, the control unit 40 provides feedback control of the auxiliary circuit 52, for example by controlling conditioning, calibration and/or recalibration of the analyte sensor 51.

In this example, the control unit 40 is configured to condition, calibrate and/or recalibrate the analyte sensor 51, when the first extracorporeal blood sensor unit 50 is arranged in the second arrangement.

In this example, the control unit 40 is configured to control the first extracorporeal blood sensor unit 50 to perform one or more checks, for example upon start up (i.e. during initialization) and/or periodically, for example to verify operation of the analyte sensor 51 such as by sensing quality control check solutions.

In this example, the control unit 40 is configured to control, for example adjust, a composition of the dialysate, for example during haemodialysis of a patient, for example by: subsequent to initiating haemodialysis of the patient, obtaining, by the control unit 40 from the first extracorporeal blood sensor unit 50, a measurement of a concentration of an analyte, for example an electrolyte, in the patient's blood; determining, by the control unit 40, whether the obtained measurement is within a predefined range; in response to determining that the measurement is not within the predefined range, determining, by the control unit 40 and based on the obtained measurement, at least one first adjustment value for adjusting a composition of the dialysate, wherein the composition of the dialysate is based on respective amounts of chemicals dispensed from a plurality of chemical sources by the dialysate circuit 30; and controlling, by the control unit 40 and based on the at least one first adjustment value, the dialysate circuit 30 to adjust the composition of the dialysate during haemodialysis for the patient by changing one or more of the respective amounts of chemicals dispensed from the plurality of chemical sources.

In this example, the control unit 40 is configured to control, based on the at least one first adjustment value, the dialysate circuit 30 to adjust the composition of the dialysate by providing one or more first instructions to direct the dialysate circuit 30 to adjust the composition of the dialysate. In this example, the control unit 40 is configured to, subsequent to adjusting the composition of the dialysate during haemodialysis based on the at least one first adjustment value, obtain a second measurement of the concentration of the analyte, for example an electrolyte, in the extracorporeal blood from the first extracorporeal blood sensor unit 50s; determine whether the at least one first adjustment value caused the second measurement to be within the predefined range; and in response to determining that the second measurement is not within the predefined range, control the dialysate circuit 30 to adjust the composition of the dialysate during haemodialysis based on the second measurement.

In this example, the control unit 40 is configured to control the dialysate circuit 30, in response to determining that the second measurement is within the predefined range, maintain the composition of the dialysate during haemodialysis.

In this example, the control unit 40 is configured to obtain a second measurement of a second concentration of a second analyte, for example an electrolyte, in the patient's blood, wherein the second analyte, for example an electrolyte, and the first analyte, for example an electrolyte, are different analytes, for example different electrolytes; determine whether the second measurement is within a second predefined range; in response to determining that the second measurement is not within the second predefined range, determine, based on the second measurement, at least one second adjustment value for adjusting the composition of the dialysate, and control the dialysate circuit 30 to adjust the composition of the dialysate based on the at least one first adjustment value and the at least one second adjustment value.

In this example, the control unit 40 is configured to control the dialysate circuit 30 to adjust the composition of the dialysate by generating actuating signals for changing the composition of the dialysate during haemodialysis based on the at least one first adjustment value; and provide the actuating signals to one or more actuators of the dialysate circuit 30 to change proportions of the respective amounts of chemicals dispensed from the plurality of chemical sources.

In this example, the control unit 40 is configured to control the dialysate circuit 30 based on determining the at least one first adjustment value the analyte, for example an electrolyte, is an increase, by generating a first actuating signal for dispensing a higher proportion of a respective chemical of a respective chemical source of the plurality of chemical sources; and based on determining the at least one first adjustment value the analyte, for example an electrolyte, is a decrease, by generating a second actuating signal for dispensing a lower proportion of the respective chemical of the respective chemical source of the plurality of chemical sources.

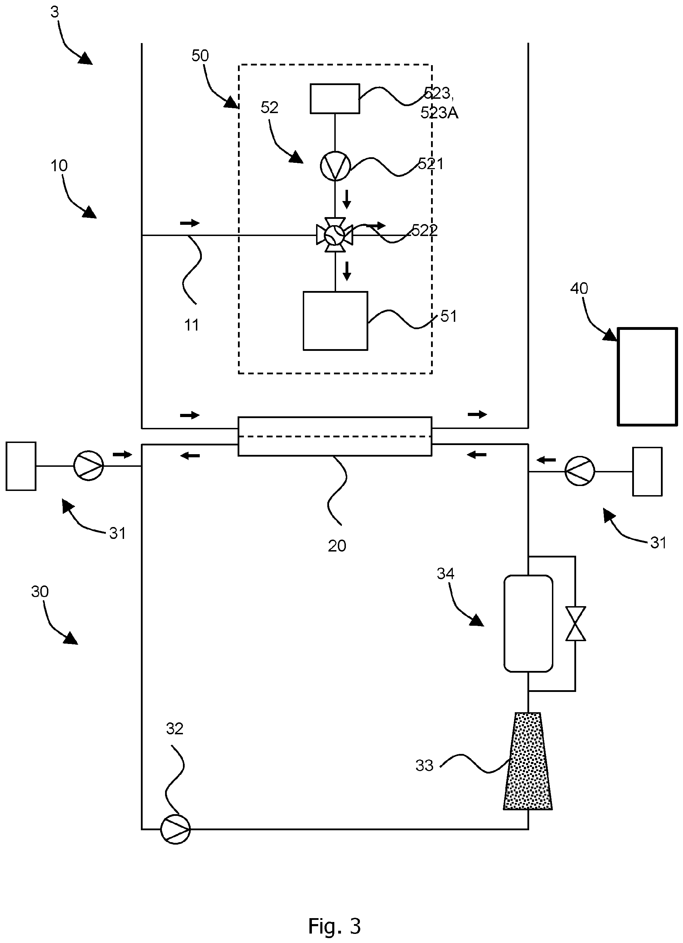

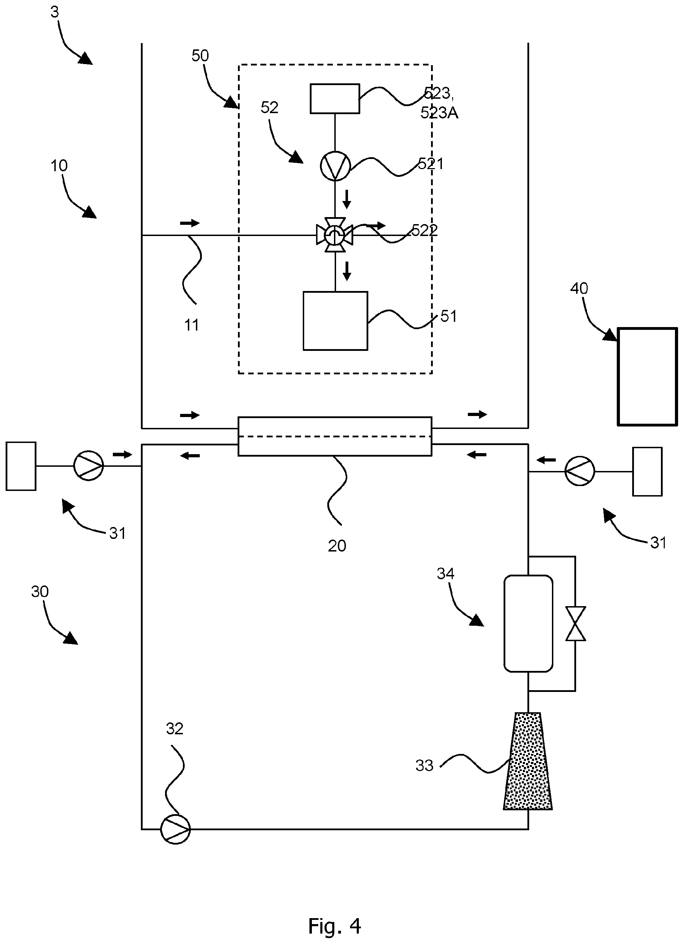

Figure 3 schematically depicts a haemodialysis machine 3 according to an exemplary embodiment, arranged in a first arrangement. Figure 4 schematically depicts the haemodialysis machine 3 of Figure 2, arranged in a second arrangement.

The haemodialysis machine 3 is generally as described with respect to the haemodialysis machine 1, description of which is not repeated for brevity.

In this example, the valve 522 is a multi-port and/or multi-way switch valve, for example wherein respective ports are fluidically coupled to the extracorporeal blood circuit 10, to the set of solution reservoirs including the first solution reservoir, to the analyte sensor 51 and to a waste, wherein the first extracorporeal blood sensor unit 50 is arranged to move between the first arrangement and the second arrangement(s) by switching the valve.

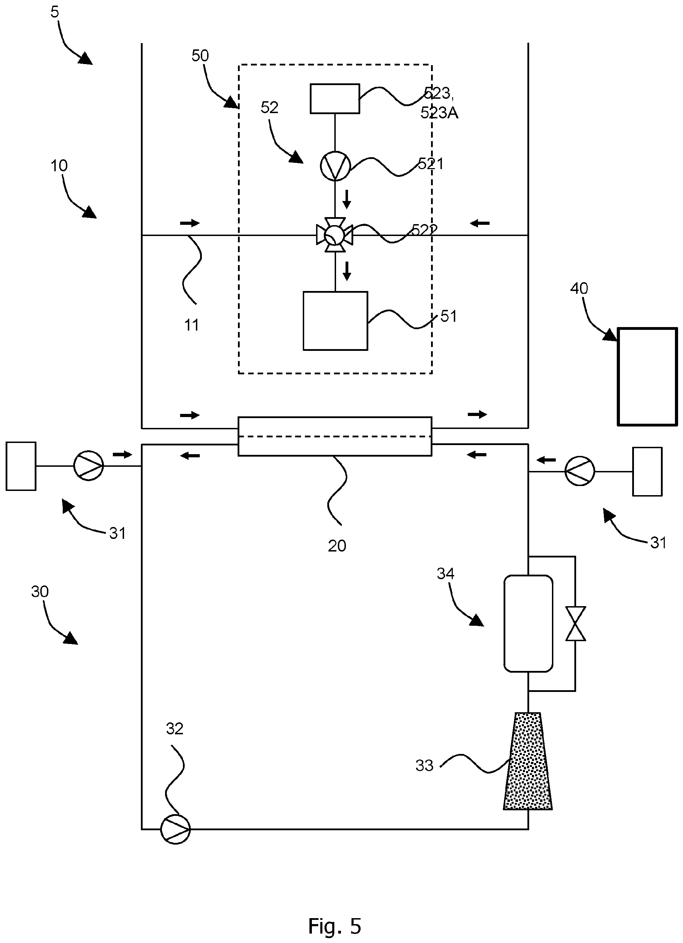

Figure 5 schematically depicts a haemodialysis machine 5 according to an exemplary embodiment, arranged in a first arrangement.

The haemodialysis machine 5 is generally as described with respect to the haemodialysis machine 1, description of which is not repeated for brevity.

In this example, the first extracorporeal blood sensor unit 50 comprises and/or is a multiplexed sensor unit, wherein the multiplexed sensor unit is arrangeable in:

the first arrangement, wherein the analyte sensor 51 is in fluid communication with the extracorporeal blood circuit 10 upstream of the dialyser 20;
the second arrangement, wherein the analyte sensor 51 is in fluid communication with the auxiliary circuit 52;
a third arrangement, wherein the analyte sensor 51 is in fluid communication with the extracorporeal blood circuit 10 downstream of the dialyser 20;
optionally, a fourth arrangement, wherein the analyte sensor 51 is in fluid communication with the dialysate circuit 30 upstream of the dialyser 20; and
optionally, a fifth arrangement, wherein the analyte sensor 51 is in fluid communication with the dialysate circuit 30 downstream of the dialyser 20.

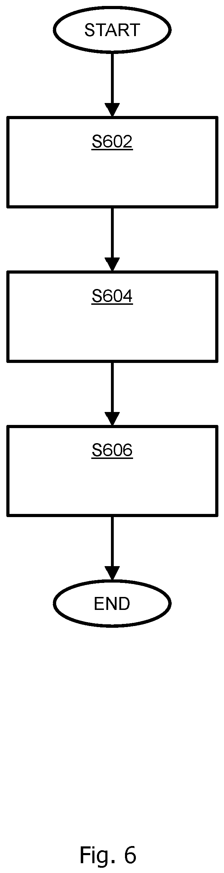

Figure 6 schematically depicts a method according to an exemplary embodiment.

The method is of controlling a haemodialysis machine. The haemodialysis machine comprises: an extracorporeal blood circuit, a dialyser, a dialysate circuit and a control unit, wherein the extracorporeal blood circuit and the dialysate circuit are respectively in fluid communication with the dialyser; and a first extracorporeal blood sensor unit comprising an analyte sensor, configured to provide signals responsive to sensing of analytes, and an auxiliary circuit, wherein the first extracorporeal blood sensor unit is fluidically coupled to the extracorporeal blood circuit. At S602, the method comprises arranging the first extracorporeal blood sensor unit in a first arrangement, wherein the analyte sensor is in fluid communication with the extracorporeal blood circuit.

At S604, the method comprises arranging the first extracorporeal blood sensor unit in a second arrangement, wherein the analyte sensor is in fluid communication with the auxiliary circuit.

At S606, the method comprises controlling, by the control unit, the dialysate circuit based, at least in part, on a first set of signals, including a first signal, received from the first extracorporeal blood sensor unit, provided by the analyte sensor when the first extracorporeal blood sensor unit is arranged in the first arrangement.

The method may include any of the steps described with respect to the fourth aspect.

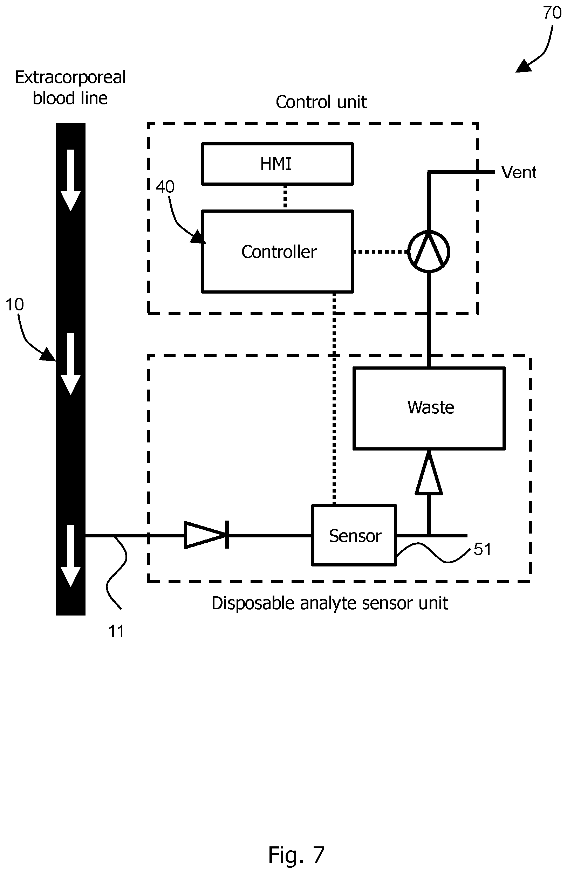

Figure 7 schematically depicts a sensor unit 70 according to an exemplary embodiment, as described with respect to the third aspect (not claimed).

The sensor unit 70 is generally as described with respect to the first extracorporeal blood sensor unit 50, description of which is not repeated for brevity.

In contrast to the first extracorporeal blood sensor unit 50, the sensor unit 70 is not arrangeable in a second arrangement and the analyte sensor 71 is in continuous flowing communication with the extracorporeal blood circuit in the first arrangement.

Continuous measurements were carried out by immersing an electrode into a fluid cell containing (volume 3 cm$^3$) relevant electrolyte solution. A stirrer bar was placed on the bottom of the beaker. The concentration of the solution was increased in intervals by the addition of 1 μL of concentrated solution to the cell followed by stirring. Concentration was decreased by the removal and replacement of the solution in the titration cell with solution of the required concentration.

Potentiostat used: EmStat product of Palm-Sens BV.

Baseline Correction was carried using the Multi-Trace software.

Curve Operations > valley to valley

Using the selection of two points on the curve in order to produce a straight line which act as a baseline.

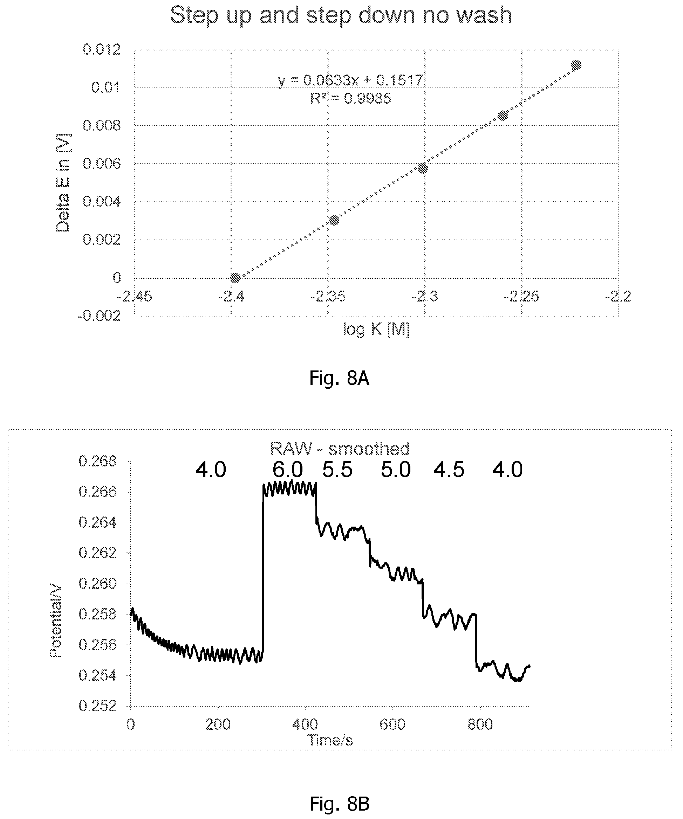

Figure 8A is a graph of Delta E (mV) as a function of log [K$^+$] (M) for the sensor unit of Figure 7; and Figure 8B is a graph of Potential (V) (smoothed) for stepping down [K$^+$] as a function of time. Particularly, Delta E (mV) increases linearly as function of log [K$^+$] (M), wherein Delta E = 0.0633log [K$^+$] + 0.1517 and the product moment correlation coefficient R$^2$ = 0.9985. The [K$^+$] was initially 4 mM, stepped up to 6 mM and then stepped down by 0.5 mM steps to 4 mM, by dilution. All concentrations in mM unless specified otherwise.

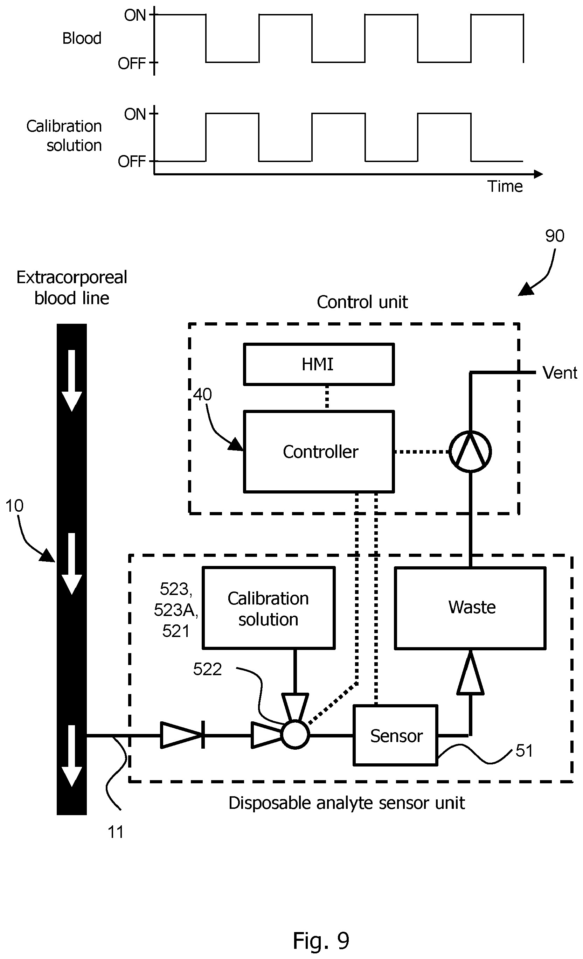

Figure 9 schematically depicts a sensor unit 90 according to an exemplary embodiment. The sensor unit 90 is generally as described with respect to the first extracorporeal blood sensor unit 50, description of which is not repeated for brevity.

In this example, the first extracorporeal blood sensor unit 90 is configured to periodically, at a predetermined frequency and for a predetermined duration, move between the first arrangement and the second arrangement(s), as schematically depicted by the control signals for the extracorporeal blood and the calibration solution, using the valve 522.

Periodic measurements were performed in a titration cell with a Teflon stir bar where solutions concentration was changed by replacing buffer with potassium solution or by replacing K$^+$ solution with buffer. The solution was gently mixed after each change in composition.

Potentiostat used: EmStat product of Palm-Sens BV.

Baseline Correction was carried using the Multi-Trace software.

## Curve Operations > valley to valley

Using the selection of two points on the curve in order to produce a straight line which act as a baseline.

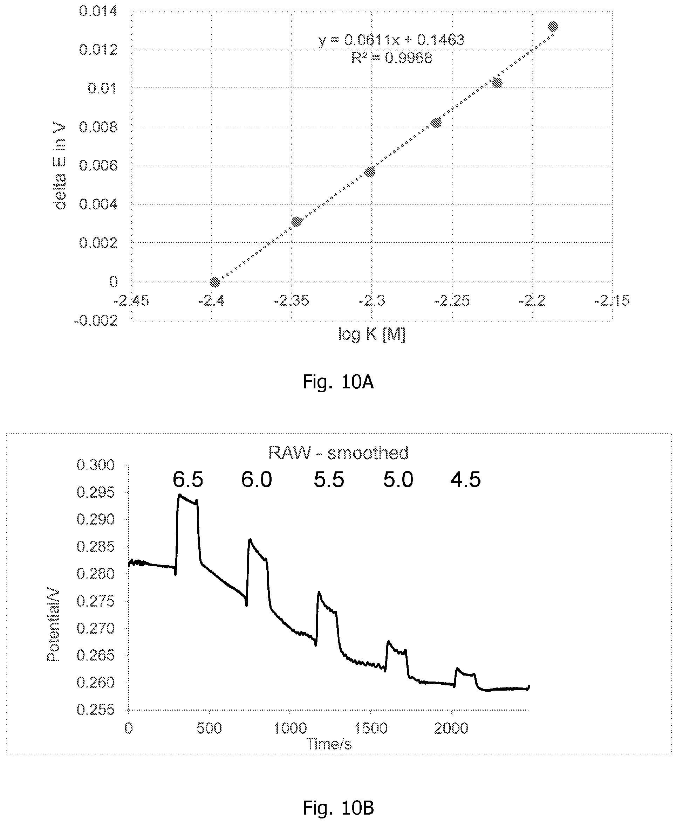

Figure 10A is a graph of Delta E (mV) as a function of log [K+] (M) for the sensor unit of Figure 9; and Figure 10B is a graph of Potential (V) (smoothed) for stepping down [K+] as a function of time. Particularly, Delta E (mV) increases linearly as function of log [K+] (M), wherein Delta E = 0.0611 log [K+] + 0.1463 and the product moment correlation coefficient $R^2$ = 0.9968.

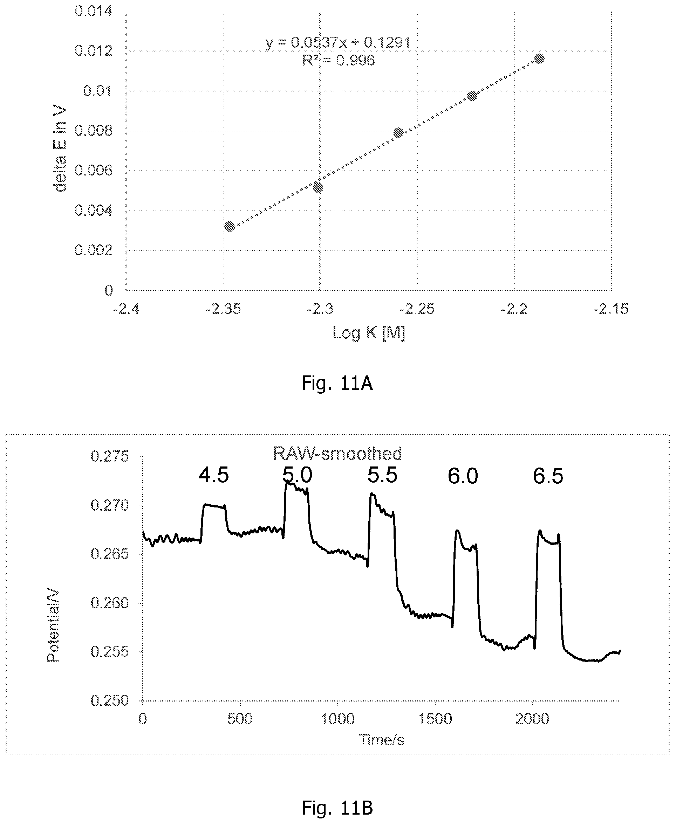

Figure 11A is a graph of Delta E (mV) as a function of log [K+] (M) for the sensor unit of Figure 9; and Figure 11B is a graph of Potential (V) (smoothed) for stepping up [K+] as a function of time. Particularly, Delta E (mV) increases linearly as function of log [K+] (M), wherein Delta E = 0.0537x + 0.1291 and the product moment correlation coefficient $R^2$ = 0.996.

### *Continuous dialysate solution monitoring over extended durations*

#### *Calibration*

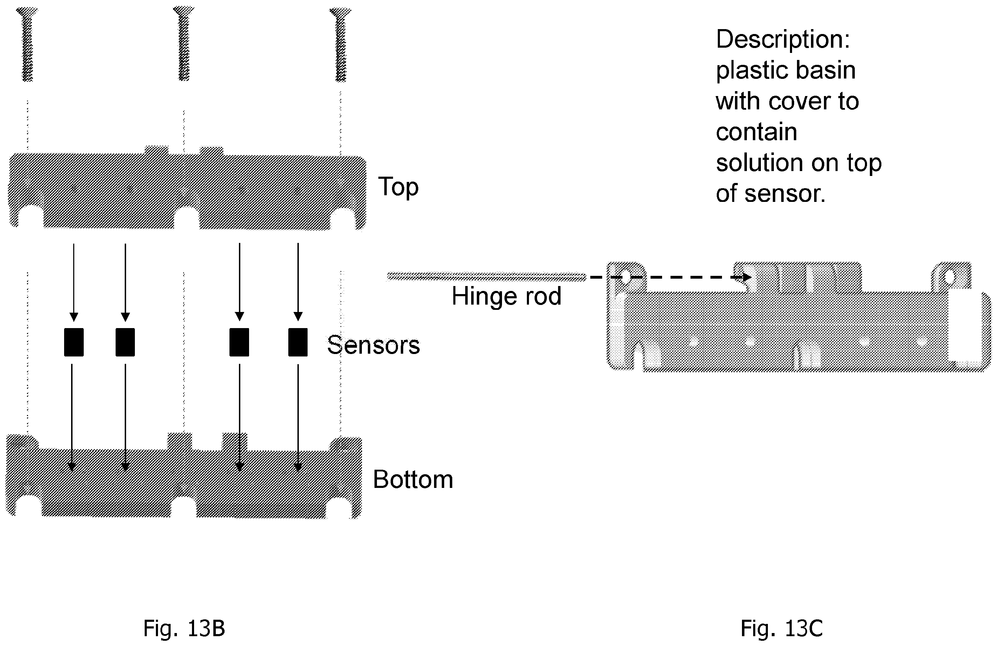

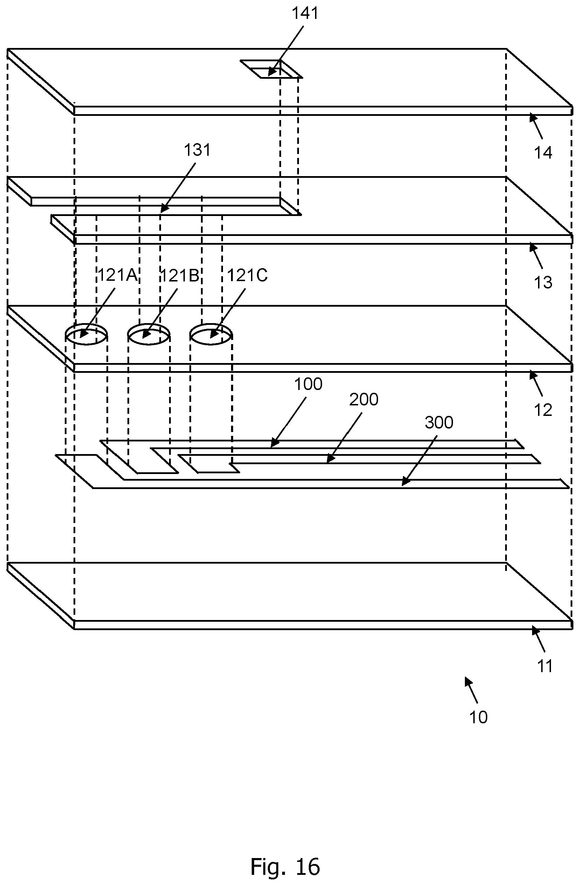

Calibration was performed using the three sensors, providing three replicates, using a protocol described below. Three sensors, generally as described with respect to Figure 16, were inserted into a custom flow cell, as described with respect to Figure 13A and 13B. The flow cell was flooded with 4mM potassium ion dialysate solution made up according to standard instructions with potassium ion concentration adjusted using potassium chloride (Mixing ratios of the dialysate are Acid Concentrate for Heamodyalisis (AC-F 119/5): $NaHCO_3$ : Ultra Pure Water of 1 : 1.58 : 42.43). The sensors were soaked for 25 minutes and the solution was replaced with 0.5mM potassium ion buffered solution (calibration solution). After a 2 minutes soaking interval, the Open Circuit Potential (OCP) response of the sensors was measured for 60 seconds (sampling interval= 0.2s) using a Mutli-Trace potentiostat (PalmSens). The calibration solution was replaced with fresh 4mM potassium ion dialysate solution and the OCP response was recorded after a 2 minutes soaking interval. The sensors were soaked for a further 10 minutes in 4mM potassium ion dialysate solution, before the solution was replaced with calibration solution and OCP response was recorded after a 2 minute soaking interval. The calibration solution was removed and 3.75mM potassium ion dialysate solution was added. The OCP response was recorded after a 2 minute soaking interval. The sensors were soaked for further 10 minutes. The described steps were repeated with dialysate solution of potassium ion concentrations 3.5, 3.25, 3.0, 2.75, 2.5, 2.25, 2.0, 1.75, 1.5, 1.25, 1.0 mM. This potassium ion concentration range is typical for potassium ion dialysate solution.

#### *Measurement*

The experiment was repeated using four fresh (i.e. new) sensors on a subsequent day, for comparison with the calibration.

#### *Results*

The results are shown in Figure 12A and Figure 12B. This protocol mimics monitoring of a solution over a long period of time and hence may be referred to as continuous measurement of K+. Note that the solution does not flow over the sensors during the measurement but is instead stationary.

Figure 12A is a semi-log calibration graph of measured OCP (V) as a function of [K+] (mM) for buffered calibration solutions, for [K+] from 4 mM to 1 mM at 0.25 mM intervals and measured simultaneously for 3 replicates (i.e. 3 sensors) at each concentration using the flow cell of Figures 13A to 13C (x-axis scale is a log10 scale). Particularly, the OCP (V) for the buffered calibration solutions increases linearly as function of log [K+] (M), wherein OCP (V) = 0.06077 [K+] (mM) + 0.007488 and the product moment correlation coefficient $R^2$ = 0.9816. Figure 12B is a graph of actual (i.e. prepared concentration of testing solution) [K+] (mM) as a function of measured [K+] (mM), for [K+] from 4 mM to 1 mM at 0.25 mM intervals and measured simultaneously for 4 replicates (i.e. 4 sensors) at each concentration using the flow cell of Figures 13A and 13B, by continuous measurement. Particularly, the measured and actual [K+] (mM) are in excellent agreement, wherein actual [K+] (mM) = 0.9626 measured [K+] (mM) + 0.04821 and the product moment correlation coefficient $R^2$ = 0.9914.

Figure 13A is a CAD exploded, perspective view from above of a flow cell for four sensors, showing one sensor in position; Figure 13B is a CAD exploded, perspective view from above of a flow cell for four sensors, showing four sensors; and Figure 13C is a CAD partially-assembled, perspective view from above the flow cell (hinge rod to be inserted). Briefly, the sensors are held between the top and the bottom of the flow cell. Respective passageways through the top of the flow cell expose the respective ISEs, CEs REs of the sensors to the solutions.

### *Continuous blood monitoring over extended durations*

#### *Calibration and Measurement of blood*

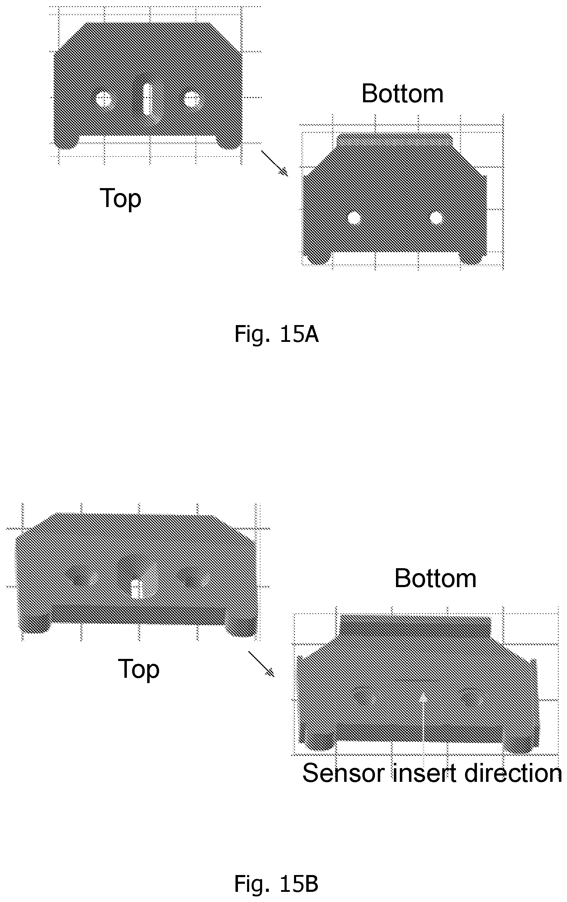

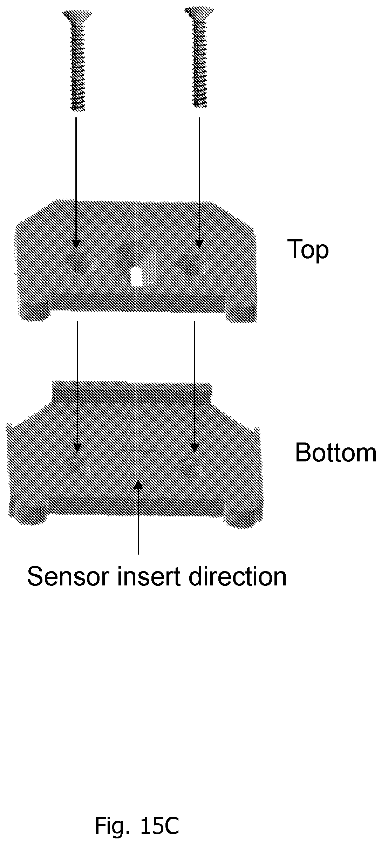

Two sensors, generally as described with respect to Figure 16, were inserted into an open top custom single channel flow cell, as described with respect to Figure 15A, Figure 15B and Figure 15C. The flow cells were flooded with 0.5mM potassium ion buffered solution (calibration solution). The sensors were soaked for 1 hour 10 minutes and the solution was replaced with fresh calibration solution. The Open Circuit Potential (OCP) response of the sensors was measured for 90 seconds (sampling interval= 0.2s) after a 2 minute soaking interval using two EmStat Pico potentiostat (PalmSens). The calibration solution was replaced with blood (generally: whole, human, fresh from a single patient), which was adjusted to potassium ion concentration of 6.8mM using potassium nitrate. OCP response was recorded after a 2 minute soaking interval. The blood was replaced with calibration solution and the sensors were soaked for a further 45 minutes, before fresh calibration solution was applied. The OCP response was recorded after a 2 minute soaking interval. The calibration solution was replaced with blood with potassium ion concentration of 5.3mM and the OCP response was recorded after a 2 minute soaking interval. The blood was once again replaced by calibration solution and soaked for 45 minutes. The procedure was repeated using blood with potassium ion concentration of 3.8 mM.

### Results

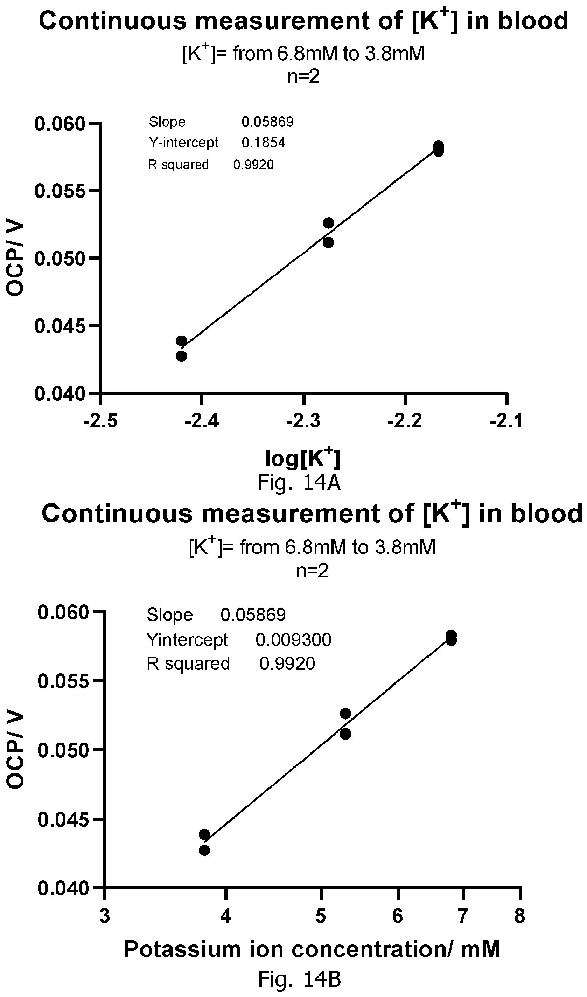

The results are shown in Figure 14A and Figure 14B. This protocol mimics monitoring of a solution over a long period of time and hence may be referred to as continuous measurement of $K^+$. Note that the solution does not flow over the sensors during the measurement but is instead stationary.

Figure 14A and Figure 14B are graphs of OCP (V) as a function of $[K^+]$ for $K^+$ in blood, for $[K^+]$ from 6.8 mM to 3.8 mM for 3 concentrations and measured for 2 replicates at each concentration using the flow cell of Figures 15A to 15C, by continuous measurement; Figure 14A is a graph of OCP (V) as a function of log $[K^+]$; and Figure 14B is a semi-log graph of the same data as Figure 14B of OCP (V) as a function of $[K^+]$ (mM) (x-axis scale is a log10 scale). Particularly, the OCP (V) for the blood increases linearly as function of log $[K^+]$, wherein OCP (V) = 0.05869 log $[K^+]$ + 0.1854 and the product moment correlation coefficient $R^2$ = 0.9920. Particularly, the OCP (V) for the blood increases linearly as function of $[K^+]$ (mM), wherein OCP (V) = 0.05869 log $[K^+]$ (mM) + 0.009300 and the product moment correlation coefficient $R^2$ = 0.9920.

Figure 15A is a CAD exploded plan view of a flow cell for two sensors; Figure 15B is a CAD exploded view from above of the flow cell; and Figure 15C is a CAD exploded view from above of the flow cell. Briefly, the sensors are held between the top and the bottom of the flow cell. Respective passageways through the top of the flow cell expose the respective ISEs, CEs REs of the sensors to the solutions and/or blood.

Figure 16 schematically depicts an exploded, perspective view of an ion-selective electrode cell (i.e. a sensor) for use in the flow cell of Figures 13A and 13B and the flow cell of Figures 15A to 15C, as described in WO/2020/193974 A1.

In more detail, Figure 16 schematically depicts an exploded, perspective view of an ion-selective electrode cell. In this example, the ion-selective electrode cell 10 comprises an ISE 100 and a reference electrode, RE, 200, together with a counter electrode, CE, 300 provided by screen printing on a rectangular substrate layer 11. Each electrode is L-shaped, including a track, provided by the long leg of the L, extending to a first end of the substrate layer 11 for coupling to a potentiostat, for example. Three corresponding circular apertures 121A, 121B, 121C are provided in a mask layer 12 that overlays the ISE 100, the RE 200 and the CE 300, thereby revealing circular portions of these respective electrodes, particularly in the short leg of the respective L. A channel layer 13 overlays the mask layer 12, having therein a channel 131 that extends from a second end of the channel layer 13, distal to the first end of the substrate layer, towards an opposed first end of the channel layer 13, whereby the circular apertures 121A, 121B, 121C are fully within the channel 131. A cover layer 14 overlays the channel layer 13 and includes a square aperture 141 coincident with the end of the channel 131. The ion-selective electrode cell 10 for sensing $K^+$ was provided as described below.

For the calibrations and measurements described with respect to Figures 12A to 15C, the sensors did not include the channel layer 13 or the cover layer 14.

### Electrodes

The ISE 100 and a CE 300 were provided, at least in part, by screen printing using commercially available carbon inks, obtained from DuPont (RTM) (Ink A: BQ242) and Gwent (RTM) (Ink B: C2110602D4) and Loctite (Ink C: EDAG PF 407A E&C), onto a polymeric substrate, particularly PE. The RE 200 was provided by screen printing using commercially available Ag/AgCl ink, obtained from Gwent Group (C2130809D5), onto the polymeric substrate. The ISE 100, the CE 300 and the RE 200 were coated with a commercially available dielectric ink. The ISE 100, the CE 300 and the RE 200 each had diameters of 1 mm and hence exposed surface areas of 0.785 $mm^2$.

### Electrode functionalisation

Polyvinyl chloride (PVC) 31.3%, bis(2-ethylhexyl) sebacate (DOS) 62.3%, potassium tetrakis(4-chlorophenyl)borate (KTpClPB) 1.4% and valinomycin 5% were mixed in cyclohexanone, to provide a polymer mix. These chemicals were obtained from Sigma-Aldrich (RTM). Particularly, 66 mg PVC, 143 $\mu$L DOS, 3 mg tetrakis(4-chlorophenyl)borate and 10 mg valinomycin were mixed in 1 mL cyclohexanone. 0.3 $\mu$l of the polymer mix was deposited on the screen printed carbon for the ISE, to provide a polymer layer having a thickness of about 10 $\mu$m to 20 $\mu$m. The ion-selective electrode cells were dried on a hotplate at 50 °C for 2 hours, transferred to an airtight container with desiccant and stored in the dark until use. Alternatively, the ion-selective electrode cells may be air-dried.

Attention is directed to all papers and documents which are filed concurrently with or previous to this specification in connection with this application and which are open to public inspection with this specification.

The invention is not restricted to the details of the foregoing embodiment(s), but it is defined by the appended claims.

## Claims

1. A haemodialysis machine (1) comprising:

   an extracorporeal blood circuit (10), a dialyser (20, a dialysate circuit (30) and a control unit (40), wherein the extracorporeal blood circuit (10) and the dialysate circuit (30) are respectively in fluid communication with the dialyser (20); and
   a first extracorporeal blood sensor unit (50, 70, 90) comprising an analyte sensor (51, 71), configured to provide signals responsive to sensing of analytes, and an auxiliary circuit (52), wherein the auxiliary circuit comprises a pump (521), a valve (522) and a set of solution and/or solvent reservoirs (523) including a first solution and/or solvent reservoir, and wherein the first extracorporeal blood sensor unit (50) is fluidically coupled to the extracorporeal blood circuit (10); wherein the first extracorporeal blood sensor unit, by switching the valve (522), is arrangeable in:

      a first arrangement,

         wherein the analyte sensor (51) is in fluid communication with the extracorporeal blood circuit (10) and
         wherein the analyte sensor (51) provides a first set of signals when the first extracorporeal blood sensor unit is in the first arrangement; and

      a second arrangement,

         wherein the analyte sensor (51) is in fluid communication with the auxiliary circuit,
         wherein the analyte sensor (51) provides a second set of signals when the first extracorporeal blood sensor unit is in the second arrangement, and

   wherein the auxiliary circuit is configured to provide a set of solutions and/or solvents, to the analyte sensor;
   wherein the control unit (40) is configured to

      control the dialysate circuit (30) based, at least in part, on the first set of signals received from the first extracorporeal blood sensor unit;
      control the auxiliary circuit (52), based at least in part on the second set of signals received from the extracorporeal blood sensor unit; and
      condition, calibrate and/or recalibrate the analyte sensor, when the first extracorporeal blood sensor unit is arranged in the second arrangement.

2. The haemodialysis machine according to claim 1, wherein the control unit (40) is configured to control the first extracorporeal blood sensor unit to periodically perform one or more checks.

3. The haemodialysis machine according to any previous claim, wherein the first extracorporeal blood sensor unit (50, 70, 90) comprises a temperature sensor for temperature-controlled recalibration of the analyte sensor and/or a flow sensor for flow-controlled recalibration of the analyte sensor.

4. The haemodialysis machine according to any previous claim, wherein the first extracorporeal blood sensor unit (50, 70, 90) is configured to periodically or intermittently move between the first arrangement and the second arrangement.

5. The haemodialysis machine according to any previous claim, wherein the analyte sensor (51) comprises an ion-selective electrode cell comprising a reference electrode, a set of ion-selective electrodes (100), including a first ion-selective electrode, and optionally a counter electrode (300).

6. The haemodialysis machine according to any previous claim, wherein the analyte sensor (51) is replaceably received in the first extracorporeal blood sensor unit (50).

7. The haemodialysis machine according to any previous claim, wherein the analyte sensor is in fluid communication with only the extracorporeal blood circuit in the first arrangement and/or wherein the analyte sensor is in fluid communication with only the auxiliary circuit in the second arrangement.

8. The haemodialysis machine according to any previous claim, wherein the analyte sensor is in flowing fluid communication with the extracorporeal blood circuit in the first arrangement.

9. The haemodialysis machine according to any previous claim, wherein the first extracorporeal blood sensor unit is fluidically coupled to the extracorporeal blood circuit upstream or downstream of the dialyser and wherein the haemodialysis machine further comprises a second extracorporeal blood sensor unit fluidically coupled to the extracorporeal blood circuit downstream or upstream of the dialyser, respectively.

10. The haemodialysis machine according to claim 9, wherein the control unit is configured to control the dialysate circuit based, at least in part, on a difference between the first set of signals received from the first extracorporeal blood sensor unit and a third set of signals received from the second extracorporeal blood sensor unit.

11. The haemodialysis machine according to any previous claim, comprising:

a first dialysate sensor unit comprising an analyte sensor, wherein the first dialysate sensor unit is fluidically coupled to the dialysate circuit, downstream or upstream of the dialyser and
a second dialysate sensor unit comprising an analyte sensor, wherein the second dialysate sensor unit is fluidically coupled to the dialysate circuit downstream or upstream of the dialyser, respectively.

12. The haemodialysis machine according to claim 11, wherein the control unit is configured to control the dialysate circuit based, at least in part, on a difference between a first set of dialysate analyte signals received from the first dialysate sensor unit and a second set of dialysate analyte signals received from the second dialysate sensor unit.

13. The haemodialysis machine according to any previous claim, wherein the dialysate circuit comprises a proportioning unit configured to provide the dialysate, a dialysate pump, a temperature sensor, a conductivity sensor and/or a dialysate regeneration unit and wherein the control unit is configured to control a composition of the dialysate.

14. The haemodialysis machine according to any previous claim, wherein the first extracorporeal blood sensor unit is configured to separate plasma from the extracorporeal blood and to direct the separated plasma to the analyte sensor.

15. The haemodialysis machine according to any previous claim, wherein the control unit comprises a trained machine learning algorithm, wherein the trained machine learning algorithm is trained to control the dialysate circuit based, at least in part, on the first set of signals received from the first extracorporeal blood sensor unit.

**Patentansprüche**

1. Hämodialysemaschine (1), umfassend:

einen extrakorporalen Blutkreislauf (10), einen Dialysator (20, einen Dialysatkreislauf (30) und eine Steuereinheit (40), wobei der extrakorporale Blutkreislauf (10) und der Dialysatkreislauf (30) jeweils in Fluidverbindung mit dem Dialysator (20) stehen; und
eine erste extrakorporale Blutsensoreinheit (50, 70, 90), umfassend einen Analytsensor (51, 71), der dazu ausgelegt ist, Signale in Reaktion auf das Erfassen von Analyten bereitzustellen, und einen Hilfskreislauf (52), wobei der Hilfskreislauf eine Pumpe (521), ein Ventil (522) und einen Satz von Lösungs- und/oder Lösungsmittelreservoirs (523) umfasst, der ein erstes Lösungs- und/oder Lösungsmittelreservoir beinhaltet, und wobei die erste extrakorporale Blutsensoreinheit (50) fluidisch an den extrakorporalen Blutkreislauf (10) gekoppelt ist;
wobei die erste extrakorporale Blutsensoreinheit, durch Umschalten des Ventils (522), wie folgt angeordnet werden kann:

in einer ersten Anordnung,
wobei der Analytsensor (51) in Fluidverbindung mit dem extrakorporalen Blutkreislauf (10) steht und
wobei der Analytsensor (51) einen ersten Satz von Signalen bereitstellt, wenn sich die erste extrakorporale Blutsensoreinheit in der ersten Anordnung befindet; und
in einer zweiten Anordnung,
wobei der Analytsensor (51) in Fluidverbindung mit dem Hilfskreislauf steht,
wobei der Analytsensor (51) einen zweiten Satz von Signalen bereitstellt, wenn sich die erste extrakorporale Blutsensoreinheit in der zweiten Anordnung befindet; und
wobei der Hilfskreislauf dazu ausgelegt ist, einen Satz von Lösungen und/oder Lösungsmitteln für den Analytsensor bereitzustellen;
wobei die Steuereinheit (40) hierzu ausgelegt ist:

Steuern des Dialysatkreislaufs (30), wenigstens teilweise basierend auf dem ersten Satz von Signalen, die von der ersten extrakorporalen Blutsensoreinheit empfangen werden;
Steuern des Hilfskreislaufs (52), wenigstens teilweise basierend auf dem zweiten Satz von Signalen, die von der ersten extrakorporalen Blutsensoreinheit empfangen werden; und
Aufbereiten, Kalibrieren und/oder erneutes Kalibrieren des Analytsensors, wenn die erste extrakorporale Blutsensoreinheit in der zweiten Anordnung angeordnet ist.

2. Hämodialysemaschine gemäß Anspruch 1, wobei die Steuereinheit (40) dazu ausgelegt ist, die erste extrakorporale Blutsensoreinheit zu steuern, um periodisch eine oder mehrere Überprüfungen durchzuführen.

3. Hämodialysemaschine gemäß einem der vorstehenden Ansprüche, wobei die erste extrakorporale Blutsensoreinheit (50, 70, 90) einen Temperatursensor zur temperaturgesteuerten Neukalibrierung des Analytsensors und/oder einen Flusssensor zur flussgesteuerten Neukalibrierung des Analytsensors umfasst.

4. Hämodialysemaschine gemäß einem der vorstehenden Ansprüche, wobei die erste extrakorporale Blutsensoreinheit (50, 70, 90) dazu ausgelegt ist, periodisch oder intermittierend zwischen der ersten Anordnung und der zweiten Anordnung zu wechseln.

5. Hämodialysemaschine gemäß einem der vorstehenden Ansprüche, wobei der Analytsensor (51) eine ionenselektive Elektrodenzelle umfasst, umfassend eine Referenzelektrode, einen Satz von ionenselektiven Elektroden (100), der eine erste ionenselektive Elektrode beinhaltet, und optional eine Zählerelektrode (300).

6. Hämodialysemaschine gemäß einem der vorstehenden Ansprüche, wobei der Analytsensor (51) austauschbar in die erste extrakorporale Blutsensoreinheit (50) aufgenommen ist.

7. Hämodialysemaschine gemäß einem der vorstehenden Ansprüche, wobei der Analytsensor, in der ersten Anordnung, nur in Fluidverbindung mit dem extrakorporalen Blutkreislauf steht und/oder wobei der Analytsensor, in der zweiten Anordnung, nur in Fluidverbindung mit dem Hilfskreislauf steht.

8. Hämodialysemaschine gemäß einem der vorstehenden Ansprüche, wobei der Analytsensor, in der ersten Anordnung, in fließender Fluidverbindung mit dem extrakorporalen Blutkreislauf steht.

9. Hämodialysemaschine gemäß einem der vorstehenden Ansprüche, wobei die erste extrakorporale Blutsensoreinheit stromaufwärts oder stromabwärts des Dialysators fluidisch an den extrakorporalen Blutkreislauf gekoppelt ist bzw. wobei die Hämodialysemaschine ferner eine zweite extrakorporale Blutsensoreinheit umfasst, die stromabwärts oder stromaufwärts des Dialysators fluidisch an den extrakorporalen Blutkreislauf gekoppelt ist.

10. Hämodialysemaschine gemäß Anspruch 9, wobei die Steuereinheit dazu ausgelegt ist, den Dialysatkreislauf wenigstens teilweise basierend auf einer Differenz zwischen dem ersten Satz von Signalen, die von der ersten extrakorporalen Blutsensoreinheit empfangen werden, und einem dritten Satz von Signalen, die von der zweiten extrakorporalen Blutsensoreinheit empfangen werden, zu steuern.

11. Hämodialysemaschine gemäß einem der vorstehenden Ansprüche, umfassend:

    eine erste Dialysatsensoreinheit, umfassend einen Analytsensor, wobei die erste Dialysatsensoreinheit stromabwärts oder stromaufwärts des Dialysators fluidisch an den Dialysatkreislauf gekoppelt ist
    bzw. eine zweite Dialysatsensoreinheit, umfassend einen Analytsensor, wobei die zweite Dialysatsensoreinheit stromabwärts oder stromaufwärts des Dialysators fluidisch an den Dialysatkreislauf gekoppelt ist.

12. Hämodialysemaschine gemäß Anspruch 11, wobei die Steuereinheit dazu ausgelegt ist, den Dialysatkreislauf wenigstens teilweise basierend auf einer Differenz zwischen einem ersten Satz von Dialysatanalytsignalen, die von der ersten Dialysatsensoreinheit empfangen werden, und einem zweiten Satz von Dialysatanalytsignalen, die von der zweiten Dialysatsensoreinheit empfangen werden, zu steuern.

13. Hämodialysemaschine gemäß einem der vorstehenden Ansprüche, wobei der Dialysatkreislauf eine Proportionierungseinheit, die dazu ausgelegt ist, das Dialysat bereitzustellen, eine Dialysatpumpe, einen Temperatursensor, einen Leitfähigkeitssensor und/oder eine Dialysatregenerierungseinheit umfasst, und wobei die Steuereinheit dazu ausgelegt ist, eine Zusammensetzung des Dialysats zu steuern.

14. Hämodialysemaschine gemäß einem der vorstehenden Ansprüche, wobei die erste extrakorporale Blutsensoreinheit dazu ausgelegt ist, Plasma von dem extrakorporealen Blut zu trennen und das abgetrennte Plasma an den Analytsensor zu leiten.

**15.** Hämodialysemaschine gemäß einem der vorstehenden Ansprüche, wobei die Steuereinheit einen trainierten Maschinenlernalgorithmus umfasst, wobei der trainierte Maschinenlernalgorithmus dafür trainiert ist, den Dialysatkreislauf wenigstens teilweise basierend auf dem ersten Satz von Signalen, die von der ersten extrakorporalen Blutsensoreinheit empfangen werden, zu steuern.

**Revendications**

**1.** Machine d'hémodialyse (1) comprenant :

un circuit sanguin extracorporel (10), un dialyseur (20), un circuit de dialysat (30) et une unité de commande (40), le circuit sanguin extracorporel (10) et le circuit de dialysat (30) étant respectivement en communication fluidique avec le dialyseur (20) ; et
une première unité de détection de sang extracorporel (50, 70, 90) comprenant un capteur d'analyte (51, 71) configuré pour fournir des signaux en réponse à la détection d'analytes, et un circuit auxiliaire (52), le circuit auxiliaire comprenant une pompe (521), une soupape (522) et un ensemble de réservoirs de solution et/ou de solvant (523) comprenant un premier réservoir de solution et/ou de solvant, la première unité de sang extracorporel (50) étant couplée fluidiquement au circuit sanguin extracorporel (10) ;
la première unité de capteur sanguin extracorporel, par commutation de la soupape (522), pouvant être agencée dans :

un premier agencement,
le capteur d'analyte (51) étant en communication fluidique avec le circuit sanguin extracorporel (10) et le capteur d'analyte (51) fournissant un premier ensemble de signaux lorsque la première unité de capteur de sang extracorporel se trouve dans le premier agencement ; et
un second agencement,
le capteur d'analyte (51) étant en communication fluidique avec le circuit auxiliaire,
le capteur d'analyte (51) fournissant un deuxième ensemble de signaux lorsque la première unité de capteur de sang extracorporel se trouve dans le second agencement, et
le circuit auxiliaire étant configuré pour fournir un ensemble de solutions et/ou de solvants au capteur d'analyte ;
l'unité de commande (40) étant configurée pour :

commander le circuit de dialysat (30) sur la base, au moins en partie, du premier ensemble de signaux reçus de la première unité de capteur sanguin extracorporel ;
commander le circuit auxiliaire (52), au moins en partie sur la base du deuxième ensemble de signaux reçus de l'unité de capteur sanguin extracorporel ; et
conditionner, étalonner et/ou réétalonner le capteur d'analyte, lorsque la première unité de capteur de sang extracorporel est agencée dans le second agencement.

**2.** Machine d'hémodialyse selon la revendication 1, l'unité de commande (40) étant configurée pour commander la première unité de capteur de sang extracorporel pour effectuer périodiquement une ou plusieurs vérifications.

**3.** Machine d'hémodialyse selon l'une quelconque des revendications précédentes, la première unité de capteur de sang extracorporel (50, 70, 90) comprenant un capteur de température pour un réétalonnage commandé en température du capteur d'analyte et/ou un capteur de débit pour un réétalonnage commandé en débit du capteur d'analyte.

**4.** Machine d'hémodialyse selon l'une quelconque des revendications précédentes, la première unité de capteur de sang extracorporel (50, 70, 90) étant configurée pour se déplacer périodiquement ou par intermittence entre le premier agencement et le second agencement.

**5.** Machine d'hémodialyse selon l'une quelconque des revendications précédentes, le capteur d'analyte (51) comprenant une cellule d'électrode sélective d'ions comprenant une électrode de référence, un ensemble d'électrodes sélectives d'ions (100), comprenant une première électrode sélective d'ions, et éventuellement une contre-électrode (300).

**6.** Machine d'hémodialyse selon l'une quelconque des revendications précédentes, le capteur d'analyte (51) étant reçu de manière remplaçable dans la première unité de capteur de sang extracorporel (50).

**7.** Machine d'hémodialyse selon l'une quelconque des revendications précédentes, le capteur d'analyte étant en communication fluidique uniquement avec le circuit sanguin extracorporel dans le premier agencement et/ou le capteur d'analyte étant en communication fluidique uniquement avec le circuit auxiliaire dans le second agencement.

8. Machine d'hémodialyse selon l'une quelconque des revendications précédentes, le capteur d'analyte étant en communication fluidique d'écoulement avec le circuit sanguin extracorporel dans le premier agencement.

9. Machine d'hémodialyse selon l'une quelconque des revendications précédentes, la première unité de capteur de sang extracorporel étant couplée fluidiquement au circuit sanguin extracorporel en amont ou en aval du dialyseur et la machine d'hémodialyse comprenant en outre une seconde unité de capteur de sang extracorporel couplée fluidiquement au circuit sanguin extracorporel en aval ou en amont du dialyseur, respectivement.

10. Machine d'hémodialyse selon la revendication 9, l'unité de commande étant configurée pour commander le circuit de dialysat sur la base, au moins en partie, d'une différence entre le premier ensemble de signaux reçus de la première unité de capteur de sang extracorporel et un troisième ensemble de signaux reçus de la seconde unité de capteur de sang extracorporel.

11. Machine d'hémodialyse selon l'une quelconque des revendications précédentes, comprenant :

une première unité de détection de dialysat comprenant un capteur d'analyte, la première unité de détection de dialysat étant couplée fluidiquement au circuit de dialysat, en aval ou en amont du dialyseur et
une seconde unité de détection de dialysat comprenant un capteur d'analyte, la seconde unité de détection de dialysat étant couplée fluidiquement au circuit de dialysat en aval ou en amont du dialyseur, respectivement.

12. Machine d'hémodialyse selon la revendication 11, l'unité de commande étant configurée pour commander le circuit de dialysat sur la base, au moins en partie, d'une différence entre un premier ensemble de signaux d'analyte de dialysat reçus de la première unité de détection de dialysat et un deuxième ensemble de signaux d'analyte de dialysat reçus de la seconde unité de détection de dialysat.

13. Machine d'hémodialyse selon l'une quelconque des revendications précédentes, le circuit de dialysat comprenant une unité de dosage configurée pour fournir le dialysat, une pompe de dialysat, un capteur de température, un capteur de conductivité et/ou une unité de régénération de dialysat et l'unité de commande étant configurée pour commander une composition du dialysat.

14. Machine d'hémodialyse selon l'une quelconque des revendications précédentes, la première unité de capteur de sang extracorporel étant configurée pour séparer le plasma du sang extracorporel et pour diriger le plasma séparé vers le capteur d'analyte.

15. Machine d'hémodialyse selon l'une quelconque des revendications précédentes, l'unité de commande comprenant un algorithme d'apprentissage automatique entraîné, l'algorithme d'apprentissage automatique entraîné étant entraîné pour commander le circuit de dialysat sur la base, au moins en partie, du premier ensemble de signaux reçus de la première unité de capteur de sang extracorporel.

Fig. 1

EP 4 436 627 B1

Fig. 2

Fig. 3

Fig. 4

Fig. 5

START

S602

S604

S606

END

Fig. 6

Fig. 7

Fig. 8A

Fig. 8B

Fig. 9

Fig. 10A

Fig. 10B

Fig. 11A

Fig. 11B

# Calibration graph

[K$^+$] from 4mM to 1mM

n=3

Slope 0.06077
Yintercept 0.007488
R squared 0.9816

Fig. 12A

# Continuous measurement of [K$^+$] in solution

[K$^+$] from 4mM to 1mM

n=4

Slope 0.9626
Y-intercept 0.04821
R squared 0.9914

Fig. 12B

Top

Sensor position
example

Bottom

Fig. 13A

Top

Sensors

Bottom

Fig. 13B

Description:
plastic basin
with cover to
contain
solution on top
of sensor.

Hinge rod

Fig. 13C

EP 4 436 627 B1

# Continuous measurement of [K⁺] in blood

[K⁺]= from 6.8mM to 3.8mM

n=2

Slope            0.05869
Y-intercept    0.1854
R squared      0.9920

log[K⁺]

Fig. 14A

# Continuous measurement of [K⁺] in blood

[K⁺]= from 6.8mM to 3.8mM

n=2

Slope       0.05869
Yintercept     0.009300
R squared        0.9920

Potassium ion concentration/ mM

Fig. 14B

Bottom

Top

Fig. 15A

Bottom

Top

Sensor insert direction

Fig. 15B

Top

Bottom

Sensor insert direction

Fig. 15C

Fig. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4662208 A
- US 9278171 B2

- WO 2020193974 A1